# EUROPEAN PATENT APPLICATION

(11) **EP 4 714 456 A2**
(43) Date of publication of application: **25.03.2026**
(21) Application number: 25227757.9
(22) Date of filing: 01.09.2021
(51) Int. Cl.: A61K 31/7105

(54) **POLYCYCLIC COMPOUNDS FOR INHIBITING RNA HELICASE DHX33 AND USE THEREOF**

(30) Priority: 16.12.2020 CN 202011487589; 23.12.2020 CN 202011532460; 30.12.2020 CN 202011609191; 01.03.2021 CN 202110225711; 29.06.2021 CN 202110724793; 29.06.2021 CN 202110724794
(62) Divisional of application: 21905132.3
(71) Applicant: Shenzhen Keye Life Technologies, Co., Ltd., Shenzhen, Guangdong 518122 (CN)
(72) Inventor: ZHANG, Yandong, Shenzhen, 518122 (CN); LI, Xianglu, Shenzhen, 518122 (CN)
(74) Representative: Perani & Partners S.p.A.

(57) **Abstract**

The present disclosure relates to a class of polycyclic compound inhibiting RNA helicase DHX33 and the application thereof. In particular, the present disclosure relates to a compound as represented by formula II or a pharmaceutically acceptable form thereof, a pharmaceutical composition comprising the same, a preparation method thereof, and a medical use thereof for preventing and/or treating DHX33-related diseases.

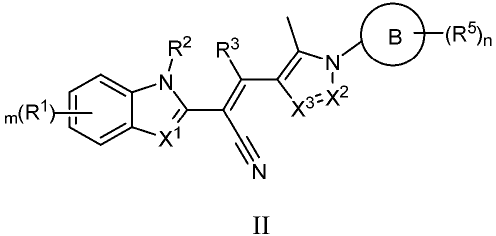

## Description

### TECHNICAL FIELD

The present disclosure belongs to the field of medicinal chemistry, and relates to a small molecule inhibitor of DHX33, a pharmaceutical composition comprising the same, a preparation method thereof and a medical use thereof for preventing and/or treating DHX33-related diseases.

### BACKGROUND

The present disclosure relates to compounds that inhibit the RNA helicase activity of DHX33. DHX33 belongs to the DEAD/H-box protein family of RNA helicases, wherein DEAD/H represents the abbreviation of the amino acid sequence, i.e., Asp-Glu-Ala-Asp/His. This sequence, together with many other conservative amino acid sequences, appears in the protein sequences of the members of the RNA helicase family and is highly involved in the binding with nucleic acid substrates and ATP hydrolysis. Although these family members have these same sequences in common, each RNA helicase has particular specificity and unique biological function. Human DHX33 protein has a molecular weight of approximately 72 kDa and has the function of unwinding nucleic acids. It utilizes the biological energy released by ATP hydrolysis to drive changes in the conformation of the complex of RNA and protein, thus participating in a variety of metabolic activities of RNA, specifically, a series of biological processes such as the transcription, splicing, editing, translation and degradation of RNA. The function of DHX33 is not merely limited to the modification of RNA molecules. Studies have demonstrated that, in addition to unwinding the two strands of RNA, DHX33 protein is also involved in the metabolism of DNA. Specifically, DHX33 protein is capable of unwinding the double-stranded structure of DNA and playing an important role in the process of gene expression.

Studies have demonstrated that, by binding to a variety of cancer-related gene promoters, DHX33 affects the methylation status of DNA and thus regulates the expression of a variety of cancer genes and the signaling pathways related to the development of tumor at genome level, which plays a crucial role in a variety of cellular activities such as the growth, proliferation, migration, apoptosis and glycometabolism of cells. In addition, it has been found that DHX33 is capable of sensing the invasion of foreign double-stranded RNA molecules and playing an important role in the innate immunity of cells. As a very important cell growth regulatory gene, DHX33 is highly expressed in a variety of cancers such as lung cancer, lymphoma, glioblastoma, breast cancer, colon cancer, liver cancer. The occurrence and development of a variety of cancers depend on the high expression of DHX33 protein. Genetic knockout of DHX33 is capable of significantly inhibiting the occurrence and development of the lung cancer driven by RAS oncogene. It has been confirmed by in-vivo and in-vitro experiments that, upon the inhibition of DHX33 protein, the occurrence and development of a variety of cancers such as breast cancer, colon cancer, brain glioma and lymphoma are inhibited significantly.

Studies have demonstrated that the function of DHX33 protein depends on its helicase activity. A DHX33 mutant lacking the helicase activity does not have the function of DHX33 protein, and the function of the wild-type DHX33 gene cannot be replaced. The present disclosure provides the structures and the synthetic methods of a variety of compounds capable of inhibiting the enzymatic activity of DHX33, and these compounds possess uses in the preparation of a drug for treating a disease or a disorder at least partially mediated by DHX33.

### SUMMARY

In the present disclosure, a series of small molecule compounds that inhibit the RNA helicase activity of DHX33 have been found through extensive studies, and these compounds have potential value in preventing and/or treating DHX33-related diseases.

In a first aspect, the present disclosure provides a compound having the structure of formula II or a pharmaceutically acceptable form thereof, 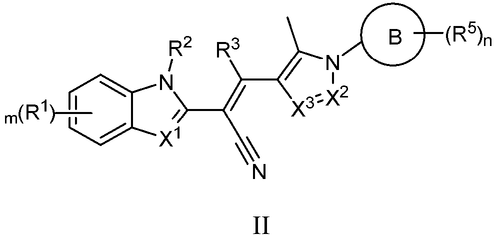 wherein
each R¹ is independently halogen, amino, -NH(C₁₋₄ alkyl), -N(C₁₋₄ alkyl)₂, nitro, C₁₋₄ alkyl, C₁₋₄ haloalkyl, C₁₋₄ alkoxy, C₁₋₄ haloalkoxy, C₁₋₄ hydroxyalkyl, -O-(C₁₋₄ alkylene)-O-(C₁₋₄ alkyl), -C(=O)-NH-(C₁₋₄ alkylene)-N(C₁₋₄ alkyl)₂, or -C(=O)-O-(C₁₋₄ alkyl), alternatively, a plurality of R¹ and the atoms to which they are attached form a 5-7 membered ring;
R² is hydrogen, C₁₋₄ alkyl, or -(C₁₋₄ alkylene)-O-(C₁₋₄ alkyl);
X¹ is N or -CR⁶;
R⁶ is hydrogen, halogen, or C₁₋₄ alkyl;
R³ is hydrogen or C₁₋₄ alkyl, alternatively, R³ and R² together with the atoms to which they are attached form a 5-6 membered ring;
X² is N or -CR⁷;
R⁷ is hydrogen, halogen, C₁₋₄ alkyl, or C₁₋₄ haloalkyl;
X³ is N or -CR⁴;
R⁴ is hydrogen, halogen, or C₁₋₄ alkyl;
B is oxazolyl, isoxazolyl, oxadiazolyl, thienyl, thiazolyl, isothiazolyl, thiadiazolyl, phenyl, or pyridyl;
each R⁵ is independently halogen, cyano, C₁₋₄ alkyl, C₁₋₄ haloalkyl, C₁₋₄ alkoxy, C₁₋₄ haloalkoxy, C₃₋₆ cycloalkyl, -C(=O)-O-(C₁₋₄ alkyl), phenyl, benzyl, pyridyl, -C(=O)-NH₂, or -NH-C(=O)-(C₁₋₄ alkyl), said phenyl, benzyl and pyridyl are optionally substituted with one or more substituents selected from halogen, cyano, amino, hydroxyl, C₁₋₄ alkyl and C₁₋₄ alkoxy;
m is 0, 1, 2, 3 or 4;
n is 0, 1, 2, 3 or 4; and
said pharmaceutically acceptable form is selected from a pharmaceutically acceptable salt, an ester, a stereoisomer, a tautomer, a solvate, a N-oxide, an isotopically labeled form, a metabolite and a prodrug.

In some embodiments, each R¹ in the above-mentioned compound of formula II or the pharmaceutically acceptable form thereof is independently fluorine, chlorine, bromine, methyl, ethyl, isopropyl, trifluoromethyl, methoxy, ethoxy, isopropoxy, trifluoromethoxy, nitro, amino, -NH(CH₃), -NH(CH₂CH₃), -N(CH₃)₂, -N(CH₂CH₃)₂, -CH₂OH, -CH₂CH₂OH, -O-(CH₂)₂-OCH₃, -C(=O)-NH-(CH₂)₂-N(CH₃)₂, -C(=O)-NH-(CH₂)₃-N(CH₃)₂, -C(=O)-OCH₃, or -C(=O)-OCH₂CH₃.

In some preferred embodiments, each R¹ in the above-mentioned compound of formula II or the pharmaceutically acceptable form thereof is independently fluorine, chlorine, bromine, methyl, trifluoromethyl, methoxy, ethoxy, isopropoxy, trifluoromethoxy, nitro, amino, -NH(CH₃), -N(CH₃)₂, -CH₂OH, -O-(CH₂)₂-OCH₃, -C(=O)-NH-(CH₂)₂-N(CH₃)₂, or -C(=O)-OCH₂CH₃.

In some more preferred embodiments, each R¹ in the above-mentioned compound of formula II or the pharmaceutically acceptable form thereof is independently fluorine, trifluoromethyl, methoxy, or trifluoromethoxy.

In some embodiments, m in the above-mentioned compound of formula II or the pharmaceutically acceptable form thereof is 0, 1, 2 or 3.

In some embodiments, R² in the above-mentioned compound of formula II or the pharmaceutically acceptable form thereof is hydrogen, methyl, ethyl, isopropyl, -(CH₂)₂-OCH₃, or -CH₂OCH₃, alternatively, R² and R³ together with the atoms to which they are attached form a 5-6 membered ring.

In some preferred embodiments, R² in the above-mentioned compound of formula II or the pharmaceutically acceptable form thereof is hydrogen, methyl, ethyl, or -CH₂OCH₃.

In some embodiments, R⁶ in the above-mentioned compound of formula II or the pharmaceutically acceptable form thereof is hydrogen, fluorine, chlorine, bromine, methyl, ethyl, or isopropyl.

In some preferred embodiments, R⁶ in the above-mentioned compound of formula II or the pharmaceutically acceptable form thereof is hydrogen or methyl.

In some embodiments, R³ in the above-mentioned compound of formula II or the pharmaceutically acceptable form thereof is hydrogen, methyl, ethyl, or isopropyl, alternatively, R³ and R² together with the atoms to which they are attached form a 5-6 membered ring.

In some preferred embodiments, R³ in the above-mentioned compound of formula II or the pharmaceutically acceptable form thereof is hydrogen or methyl.

In some embodiments, R⁷ in the above-mentioned compound of formula II or the pharmaceutically acceptable form thereof is hydrogen, fluorine, chlorine, bromine, methyl, ethyl, isopropyl, or trifluoromethyl.

In some preferred embodiments, R⁷ in the above-mentioned compound of formula II or the pharmaceutically acceptable form thereof is hydrogen or methyl.

In some embodiments, R⁴ in the above-mentioned compound of formula II or the pharmaceutically acceptable form thereof is hydrogen, fluorine, chlorine, bromine, methyl, ethyl, or isopropyl.

In some preferred embodiments, R⁴ in the above-mentioned compound of formula II or the pharmaceutically acceptable form thereof is hydrogen or methyl.

In some embodiments, B in the above-mentioned compound of formula II or the pharmaceutically acceptable form thereof is

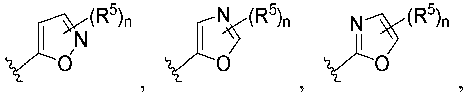

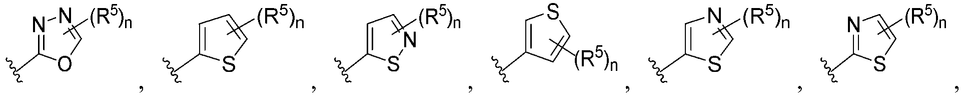

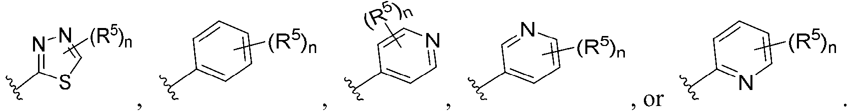

In some embodiments, each R⁵ in the above-mentioned compound of formula II or the pharmaceutically acceptable form thereof is independently fluorine, chlorine, bromine, cyano, methyl, ethyl, isopropyl, tert-butyl, cyclopropyl, cyclopentyl, cyclohexyl, trifluoromethyl, methoxy, ethoxy, isopropoxy, trifluoromethoxy, -C(=O)-OCH₃, -C(=O)-OCH₂CH₃, phenyl, benzyl, pyridyl, -C(=O)-NH₂, -NH-C(=O)-CH₃, or -NH-C(=O)-CH₂CH₃.

In some preferred embodiments, each R⁵ in the above-mentioned compound of formula II or the pharmaceutically acceptable form thereof is independently fluorine, chlorine, bromine, cyano, methyl, ethyl, isopropyl, trifluoromethyl, methoxy, trifluoromethoxy, -C(=O)-OCH₃, phenyl, benzyl, pyridyl, -C(=O)-NH₂, or -NH-C(=O)-CH₃.

In some more preferred embodiments, each R⁵ in the above-mentioned compound of formula II or the pharmaceutically acceptable form thereof is independently cyano, methyl, trifluoromethyl, methoxy, trifluoromethoxy, -C(=O)-OCH₃, or -NH-C(=O)-CH₃.

In some embodiments, n in the above-mentioned compound of formula II or the pharmaceutically acceptable form thereof is 0, 1, 2 or 3.

In some embodiments, the above-mentioned compound of formula II or the pharmaceutically acceptable form thereof is a compound of any one of formula IIa-1 to formula IIa-5 or a pharmaceutically acceptable form thereof, 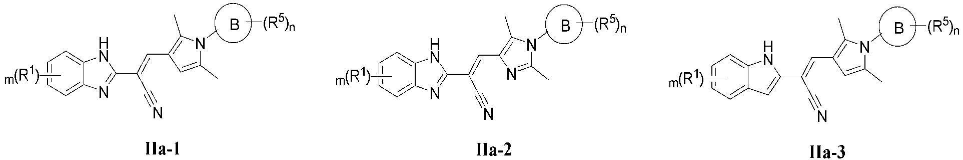 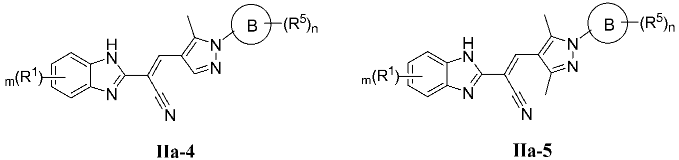 wherein B, R¹, R⁵, m and n are as defined in formula II.

It should be understood by those skilled in the art that the present disclosure encompasses compounds derived from any combination of various embodiments. Embodiments obtained by combining a technical feature or a preferred technical feature in one embodiment with a technical feature or a preferred technical feature in another embodiment are also included within the scope of the present disclosure.

In a second aspect, the present disclosure also provides the following compounds or the pharmaceutically acceptable salts, stereoisomers, tautomers, solvates, N-oxides, or isotopically labeled forms thereof:

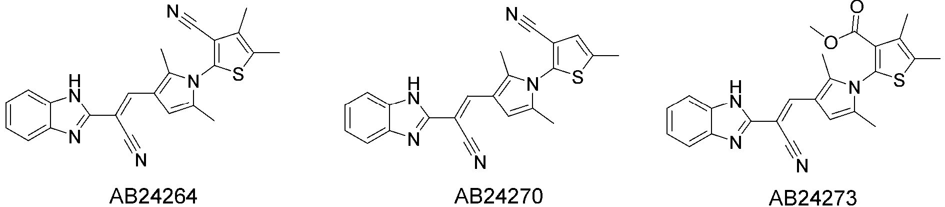

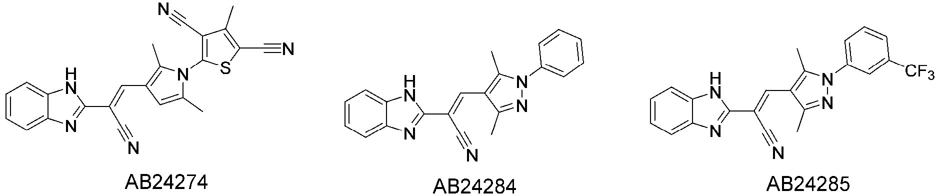

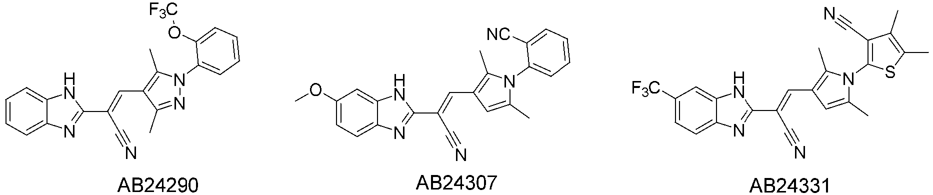

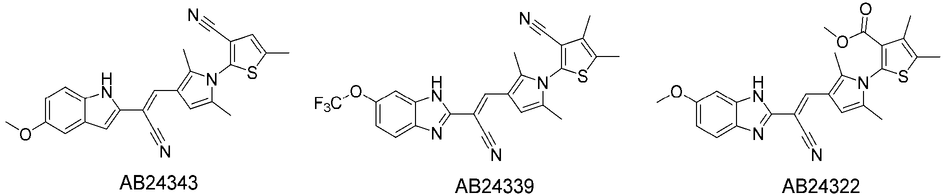

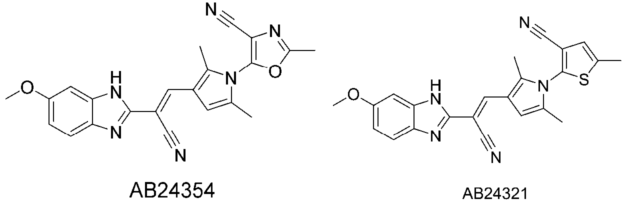

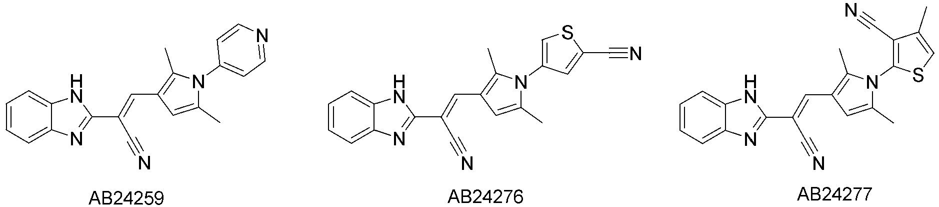

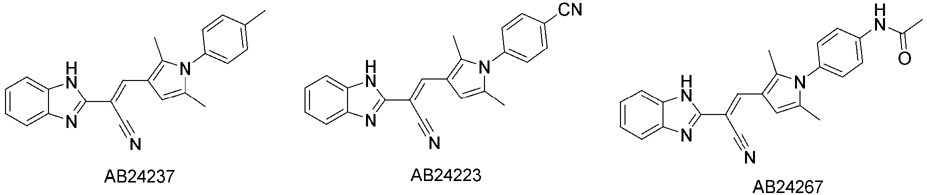

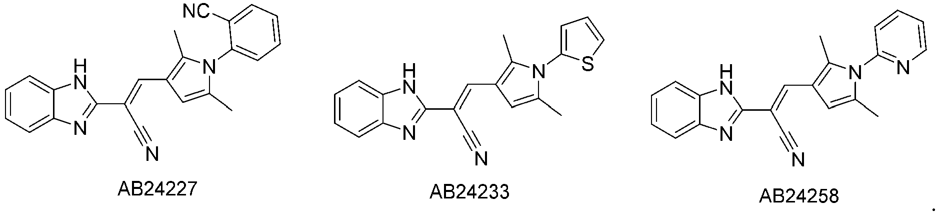

In a third aspect, the present disclosure provides a pharmaceutical composition, comprising at least one of the above-mentioned compounds of formula II or the pharmaceutically acceptable form thereof, and one or more pharmaceutically acceptable carriers.

In a fourth aspect, the present disclosure provides the above-mentioned compounds of formula II or the pharmaceutically acceptable form thereof or the above-mentioned pharmaceutical composition, for use as a DHX33 inhibitor for preventing and/or treating a disease or a disorder at least partially mediated by DHX33.

In a fifth aspect, the present disclosure provides the use of the above-mentioned compounds of formula II or the pharmaceutically acceptable form thereof or the above-mentioned pharmaceutical composition in preparation of a drug for preventing and/or treating a disease or a disorder at least partially mediated by DHX33.

In a sixth aspect, the present disclosure provides a method for preventing and/or treating a disease or a disorder at least partially mediated by DHX33, comprising the following step of administering a prophylactically and/or a therapeutically effective amount of the above-mentioned compounds of formula II or the pharmaceutically acceptable form thereof or the above-mentioned pharmaceutical composition to an individual in need thereof.

The present disclosure is not limited to the specific embodiments described herein. It should also be understood that the terms used herein are merely used for describing rather than limiting specific embodiments.

### Definition of Terms

Unless otherwise specified, the meanings of the following terms in the present disclosure are as follows.

The terms "comprise", "include", "have" or "contain" or any other variation thereof are intended to encompass non-exclusive or open-ended inclusions. For example, a composition, method or device comprising a series of elements is not necessarily limited to the elements that have been explicitly listed, and may also comprise other elements that are not explicitly listed or the elements inherent to the above-described composition, method or device.

When the lower limit and the upper limit of a numerical range are disclosed, any numerical value or any subrange falling within this range is intended to be specifically disclosed. In particular, each numerical range (for example, in the form of "about a to b", or equivalent form of "approximately a to b", or equivalent form of "about a-b") of a parameter disclosed herein should be understood to encompass each numerical value and each subrange therein. For example, "C₁₋₄" should be understood to encompass any subrange and each point value therein, e.g., C₂₋₄, C₃₋₄, C₁₋₂, C₁₋₃, C₁₋₄ and the like, as well as C₁, C₂, C₃, C₄ and the like. For another example, "5-10 membered" should be understood to encompass any subrange and each point value therein, e.g., 5-6 membered, 5-7 membered, 5-8 membered, 5-9 membered, 6-7 membered, 6-8 membered and the like, as well as 5 membered, 6 membered, 7 membered, 8 membered, 9 membered, 10 membered and the like.

The term "pharmaceutical composition" refers to a composition that may be used as a drug and comprises a pharmaceutically active ingredient (or a therapeutic agent) and alternatively one or more pharmaceutically acceptable carriers. The term "pharmaceutically acceptable carrier" refers to an excipient that is administered together with a therapeutic agent and is suitable for contacting with the tissues of human beings and/or other animals without excessive toxicity, irritation, allergic response or other problems or complications corresponding to a reasonable benefit/risk ratio within the scope of reasonable medical judgment. Pharmaceutically acceptable carriers usable in the present disclosure include but are not limited to: a) diluents; b) lubricants; c) binders; d) disintegrants; e) absorbing agents, colorants, flavoring agents and/or sweeteners; f) emulsifying agents or dispersing agents; and/or g) substances that enhance the absorption of the compound, and the like.

The above-mentioned pharmaceutical composition may act systemically and/or locally. For this purpose, they may be administered via suitable routes such as parenteral route, topical route, intravenous route, oral route, subcutaneous route, intraarterial route, intradermal route, transdermal route, rectal route, intracranial route, intraperitoneal route, intranasal route, intramuscular route, or may be administered as an inhalant.

The administration via the above-mentioned routes may be realized via suitable dosage forms. Dosage forms usable in the present disclosure include but are not limited to: tablets, capsules, lozenges, hard candies, powders, sprays, creams, ointments, suppositories, gels, pastes, lotions, ointments, aqueous suspensions, injectable solutions, elixirs, syrups, and the like.

When administered orally, the above-mentioned pharmaceutical composition may be prepared into any orally acceptable formulation, including but not limited to tablets, capsules, aqueous solutions, aqueous suspensions, and the like.

The above-mentioned pharmaceutical composition may also be administered in the form of sterile injection, including sterile injectable aqueous suspensions or oily suspensions, or sterile injectable aqueous solutions or oily solutions. Among these, usable carriers include but are not limited to water, Ringer's solution and isotonic sodium chloride solution. In addition, sterile non-volatile oils such as monoglycerides or diglycerides may also be used as a solvent or a suspending medium.

The above-mentioned pharmaceutical composition may comprise 0.01 mg to 1000 mg of at least one of the above-mentioned compounds of formula II or a pharmaceutically acceptable form thereof.

The term "a disease or a disorder at least partially mediated by DHX33" refers to a disease (such as cancer, viral infection and inflammation) of which the pathogeneses at least include DHX33-related factors in part.

The term "effective amount" refers to a dosage that is capable of inducing a cell, a tissue, an organ or an organism (e.g., an individual) to generate biological or medical response and is sufficient to achieve the desired prophylactic and/or therapeutic effects.

Dosing regimens may be adjusted to provide the optimal desired response. For example, the drug may be administered in a single dose, may be administered in divided doses over time, or may be administered in a dose that is reduced or increased proportionately according to the actual situation. It could be understood that, for any particular individual, the specific dosing regimen should be adjusted as needed and according to the professional judgment of the person administering the composition or supervising the administration of the composition.

The term "in need thereof" refers to a judgment of a physician or other nursing staff that an individual needs or will benefit from the prophylactic and/or therapeutic process, and this judgment is obtained based on various factors in the field of expertise of the physician or other nursing staff.

The term "individual" (or referred to as subject) refers to a human or a non-human animal. The individuals of the present disclosure include individuals suffering from a disease and/or disorder (patients) and normal individuals. The non-human animals of the present disclosure include all vertebrates, for example, non-mammals such as birds, amphibians and reptiles, and mammals such as non-human primates, livestock and/or domesticated animals (such as sheeps, dogs, cats, cows and pigs).

The term "treating" means alleviating or eliminating the targeted disease or disorder. If a subject receives a therapeutic amount of the compound or the pharmaceutically acceptable form thereof of the present disclosure or the pharmaceutical composition of the present disclosure and this subject exhibits observable and/or detectable remission and/or improvement in at least one indicator or symptom, it indicates that this subject has been successfully "treated". It could be understood that treatment not only includes the complete treatment, but also includes a case where the complete treatment has not been achieved while some biologically or medically relevant results have been achieved. To be specific, "treating" means that the compound or the pharmaceutically acceptable form thereof of the present disclosure or the pharmaceutical composition of the present disclosure is capable of realizing at least one of the following effects, for example, (1) preventing the occurrence of a disease in an animal that may be predisposed to the disease but have not yet experienced or exhibited the pathology or symptomatology of the disease, (2) inhibiting a disease (that is, preventing further progression of pathology and/or symptomatology) in an animal that is experiencing or exhibiting the pathology or symptomatology of the disease, and (3) ameliorating a disease (that is, reversing pathology and/or symptomatology) in an animal that is experiencing or exhibiting the pathology or symptomatology of the disease.

The term "pharmaceutically acceptable salt" refers to a salt of a compound of the present disclosure which is substantially non-toxic to an organism. Pharmaceutically acceptable salts generally include but are not limited to salts formed by the reaction of the compounds of the present disclosure with pharmaceutically acceptable inorganic/organic acids or inorganic/organic bases, and such salts are also referred to as acid addition salts or base addition salts. As for reviews of suitable salts, please refer to, for example, Jusiak, Soczewinski, et al., Remington's Pharmaceutical Sciences [M], Mack Publishing Company, 2005 and Stahl, Wermuth, Handbook of Pharmaceutical Salts: Properties, Selection, and Use [M], Wiley-VCH, 2002. A method for preparing the pharmaceutically acceptable salts of the compounds of the present disclosure is known to those skilled in the art.

The term "pharmaceutically acceptable ester" refers to an ester that is substantially non-toxic to an organism and is hydrolyzed to a compound of the present disclosure or a salt thereof in the organism. Pharmaceutically acceptable esters generally include but are not limited to esters formed by the compounds of the present disclosure and pharmaceutically acceptable carboxylic acids or sulfonic acids, and such esters are also referred to as carboxylic acid esters or sulfonic acid esters.

The term "isomers" refers to compounds that have the same molecular weight due to the same number and type of atoms while having different spatial arrangement of atoms or configurations.

The term "stereoisomer" (or referred to as "optical isomer") refers to a stable isomer that has a vertical asymmetric plane resulting from at least one chiral factor (including a chiral center, a chiral axis, a chiral plane, and the like) and thus is capable of enabling the rotation of the plane-polarized light. Since asymmetric center(s) and other chemical structures that may result in stereoisomerism exist in the compounds of the present disclosure, the present disclosure also includes these stereoisomers and the mixtures thereof. Unless otherwise indicated, all stereoisomeric forms of the compounds of the present disclosure are within the scope of the present disclosure.

The term "tautomers" (or referred to as "tautomeric forms") refers to structural isomers that have different energy and are interconvertible via a low energy barrier. If tautomerism is possible (for example, in solution), a chemical equilibrium of tautomers may be achieved. For example, proton tautomers (or referred to as proton-transfer tautomers) include but are not limited to those obtained by the interconversion via proton transfer such as keto-enol tautomerism, imine-enamine tautomerism and amide-iminol tautomerism. Unless otherwise indicated, all tautomeric forms of the compounds of the present disclosure are within the scope of the present disclosure.

The term "solvate" refers to a substance formed by the association of a compound of the present disclosure (or a pharmaceutically acceptable salt thereof) with at least one kind of solvent molecule via non-covalent intermolecular force. For example, solvates include but are not limited to hydrates (including hemihydrates, monohydrates, dihydrates, trihydrates, and the like), ethanolates, acetonates, and the like.

The term "N-oxide" refers to a compound formed by the oxidation of the nitrogen atom(s) in the structure of a tertiary amine-based compound or a nitrogen-containing (aromatic) heterocyclic compound. For example, the nitrogen atoms in the parent structure of the compound of formula I may be oxidized, so that the corresponding N-oxide may be formed.

The term "isotopically labeled form" refers to a derivative compound formed by replacing a particular atom in a compound of the present disclosure with an isotope thereof. Unless otherwise indicated, the compounds of the present disclosure comprise various isotopes of H, C, N, O, F, P, S and Cl, such as, but not limited to, ²H(D), ³H(T), ¹³C, ¹⁴C, ¹⁵N, ¹⁷O, ¹⁸O, ¹⁸F, ³¹P, ³²P, ³⁵S, ³⁶S and ³⁷Cl.

The term "metabolite" refers to a derivative compound formed by the metabolism of a compound of the present disclosure. As for further information on metabolism, please refer to Goodman and Gilman's: The Pharmacological Basis of Therapeutics (9th ed.) [M], McGraw-Hill International Editions, 1996. The present disclosure encompasses all possible forms of the metabolites of the compounds of the present disclosure, that is, substances formed in an individual to whom the compound(s) of the present disclosure is administered. The metabolites of compounds may be identified by techniques well known in the art, and the activity thereof may be characterized by assays.

The term "prodrug" refers to a derivative compound capable of directly or indirectly providing a compound of the present disclosure upon administration to an individual. Particularly preferred derivative compounds or prodrugs are compounds that are capable of improving the bioavailability of the compounds of the present disclosure (e.g., enabling easier absorption into blood) or facilitating the delivery of the parent compounds to the sites of action (e.g., the lymphatic system) when administered to an individual. Unless otherwise indicated, all forms of the prodrugs of the compounds of the present disclosure are within the scope of the present disclosure, and various forms of the prodrugs are known in the art, for example, see T. Higuchi, V. Stella, Pro-drugs as Novel Drug Delivery Systems [J], American Chemical Society, Vol. 14, 1975**.** In addition, the present disclosure also encompasses the compounds of the present disclosure that contain protecting groups. In any process of preparing the compounds of the present disclosure, it may be essential and/or desirable to protect the sensitive group(s) or reactive group(s) on any relevant molecule, and the chemically protected forms of the compounds of the present disclosure are thus formed. It could be achieved by conventional protecting groups, such as the protecting groups described in T. W. Greene, P. G. M. Wuts, Protective Groups in Organic Synthesis [M], John Wiley & Sons, 2006**.** These protecting groups may be removed at an appropriate subsequent stage using methods known in the art.

The term "each independently" means that at least two groups (or cyclic systems) that are present in the structure and have the same or similar range of values may have the same or different meanings under certain circumstances. For example, substituent X and substituent Y are each independently hydrogen, halogen, hydroxyl, cyano, alkyl or aryl, then when substituent X is hydrogen, substituent Y may be hydrogen, and may also be halogen, hydroxyl, cyano, alkyl or aryl; similarly, when substituent Y is hydrogen, substituent X may be hydrogen, and may also be halogen, hydroxyl, cyano, alkyl or aryl.

When used herein alone or in combination with other groups, the term "halogen" refers to fluorine (F), chlorine (Cl), bromine (Br) and iodine (I).

When used herein alone or in combination with other groups, the term "alkyl" refers to a linear or branched aliphatic hydrocarbon group. For example, the term "C₁₋₆ alkyl" used in the present disclosure refers to an alkyl group having 1 to 6 carbon atoms. For example, alkyl groups include but are not limited to methyl, ethyl, n-propyl, isopropyl, n-butyl, sec-butyl, isobutyl, tert-butyl, or the like. Alkyl groups may be optionally substituted or unsubstituted.

When used herein alone or in combination with other groups, the term "alkylene" refers to a linear or branched divalent saturated aliphatic hydrocarbon group, and the two groups (or fragments) attached to alkylene may be attached to either the same carbon atom or different carbon atoms. For example, the term "C₁₋₆ alkylene" used herein refers to an alkylene group having 1 to 6 carbon atoms (e.g., methylene, 1,1-ethylene, 1,2-ethylene, 1,2-propylene, 1,3-butylene, and the like). Alkylene groups may be optionally substituted or unsubstituted.

When used herein alone or in combination with other groups, the term "haloalkyl" refers to an alkyl group substituted with one or more (such as 1 to 3) same or different halogen atoms. For example, the term "C₁₋₆ haloalkyl" used in the present disclosure refers to a haloalkyl group having 1 to 6 carbon atoms. For example, haloalkyl groups include but are not limited to -CH₂F, -CHF₂, -CF₃, -CH₂CF₃, -CF₂CF₃, -CH₂CH₂CF₃, -CH₂Cl, and the like. Haloalkyl groups may be optionally substituted or unsubstituted.

When used herein alone or in combination with other groups, the term "hydroxyalkyl" refers to an alkyl group substituted with one or more (such as 1 to 3) hydroxyl groups. For example, the term "C₁₋₆ hydroxyalkyl" used in the present disclosure refers to a hydroxyalkyl group having 1 to 6 carbon atoms. For example, hydroxyalkyl groups include but are not limited to 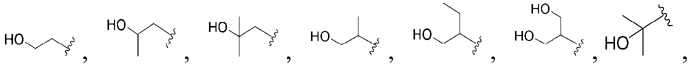 or the like. Hydroxyalkyl groups may be optionally substituted or unsubstituted.

When used herein alone or in combination with other groups, the term "alkoxy" refers to an alkyl group that is attached to the remaining part of the molecule via an oxygen atom. For example, alkoxy groups include but are not limited to methoxy, ethoxy, n-propoxy, isopropoxy, n-butoxy, isobutoxy, tert-butoxy, and the like. Alkoxy groups may be optionally substituted or unsubstituted.

When used herein alone or in combination with other groups, the term "haloalkoxy" refers to a monovalent linear or branched haloalkyl-O-group, which is substituted with at least one atom selected from fluorine, chlorine, bromine and iodine, may comprise degree(s) of unsaturation, and is attached to other groups via a single bond attached to an oxygen atom, such as C₁₋₆ haloalkoxy. For example, haloalkoxy groups include but are not limited to fluoromethoxy (-OCH₂F), difluoromethoxy (-OCHF₂), trifluoromethoxy(-OCF₃), 1-fluoroethoxy (-OCHFCH₃), 2-fluoroethoxy (-OCH₂CH₂F), 1,2-difluoroethoxy (-OCHFCH₂F), 2,2-difluoroethoxy (-OCH₂CHF₂), 1,2,2-trifluoroethoxy (-OCHFCHF₂), 2,2,2-trifluoroethoxy (-OCH₂CF₃), and the like.

When used herein alone or in combination with other groups, the term "alkylthio" refers to an alkyl group that is attached to the remaining part of the molecule via a sulfur atom. For example, alkylthio groups include but are not limited to methylthio, ethylthio, n-propylthio, isopropylthio, and the like. Alkylthio groups may be optionally substituted or unsubstituted.

When used herein alone or in combination with other groups, the term "haloalkylthio" refers to a monovalent linear or branched haloalkyl-S-group, which is substituted with at least one atom selected from fluorine, chlorine, bromine and iodine, may comprise degree(s) of unsaturation, and is attached to other groups via a single bond attached to a sulfur atom, such as C₁₋₆ haloalkylthio. For example, haloalkylthio groups include but are not limited to fluoromethylthio (-SCH₂F), difluoromethylthio (-SCHF₂), trifluoromethylthio (-SCF₃), and the like.

When used herein alone or in combination with other groups, the term "cycloalkyl" refers to a monocyclic or polycyclic (such as bicyclic) non-aromatic hydrocarbon group that is saturated or partially saturated. For example, the term "C₃₋₆ cycloalkyl" used in the present disclosure refers to a cycloalkyl group having 3 to 6 carbon atoms. For example, cycloalkyl groups include but are not limited to cyclopropyl, cyclobutyl, cyclopentyl, cyclohexyl, or the like. Cycloalkyl groups may be optionally substituted or unsubstituted.

When used herein alone or in combination with other groups, the term "heterocyclyl" refers to a monocyclic or polycyclic (for example, bicyclic such as fused cyclic, bridged cyclic or spiro cyclic) non-aromatic group that is saturated or partially saturated and has ring atoms consisting of carbon atoms and at least one heteroatom selected from N, O and S, wherein the sulfur atom is optionally substituted to form S(=O), S(=O)₂ or S(=O)(=NR^{x}) and R^{x} is independently H or C₁₋₄ alkyl. A heterocyclyl group may be attached to the remaining part of the molecule via any one of the ring atoms if the requirements of valence are satisfied. For example, the term "3-8 membered heterocyclyl" used in the present disclosure refers to a heterocyclic group having 3 to 8 ring atoms. Common heterocyclyl groups include (but are not limited to) oxiranyl, aziridinyl, azetidinyl, oxetanyl, tetrahydrofuranyl, dioxolyl, pyrrolidinyl, pyrrolidonyl, imidazolidinyl, pyrazolidinyl, tetrahydropyranyl, piperidinyl, piperazinyl, morpholinyl, thiomorpholinyl, dithianyl or trithianyl. A heterocyclic group in the present disclosure is optionally substituted with one or more substituents as described in the present disclosure.

When used herein alone or in combination with other groups, the term "aryl" refers to a monocyclic or fused polycyclic aromatic hydrocarbon group that has a conjugated system of π electrons. For example, the term "C₆₋₁₀ aryl" used in the present disclosure refers to an aryl group having 6 to 10 carbon atoms. Common aryl group include (but are not limited to) phenyl, naphthyl, anthryl, phenanthryl, acenaphthenyl, azulenyl, fluorenyl, indenyl, pyrenyl, and the like. An aryl group in the present disclosure is optionally substituted with one or more substituents as described in the present disclosure.

When used herein alone or in combination with other groups, the term "heteroaryl" refers to a monocyclic or fused polycyclic aromatic group that has a conjugated system of π electrons and has ring atoms consisting of carbon atoms and at least one heteroatom selected from N, O and S. A heteroaryl group may be attached to the remaining part of the molecule via any one of the ring atoms if the requirements of valence are satisfied. For example, the term "5-10 membered heteroaryl" used in the present disclosure refers to a heteroaryl group having 5 to 10 ring atoms. Common heteroaryl groups include (but are not limited to) thienyl, furanyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, triazinyl and the benzo derivatives thereof, pyrrolopyridyl, pyrrolopyrazinyl, pyrazolopyridyl, imidazolopyridyl, pyrrolopyrimidinyl, pyrazolopyrimidinyl, purinyl, and the like. A heteroaryl group in the present disclosure is optionally substituted with one or more substituents as described in the present disclosure (e.g., halogen, C₁₋₆ alkyl, and the like).

When used herein alone or in combination with other groups, the term "hydroxyl" refers to -OH.

When used herein alone or in combination with other groups, the term "cyano" refers to -CN.

When used herein alone or in combination with other groups, the term "amino" refers to -NH₂.

When used herein alone or in combination with other groups, the term "nitro" refers to -NO₂.

### BRIEF DESCRIPTION OF THE DRAWINGS

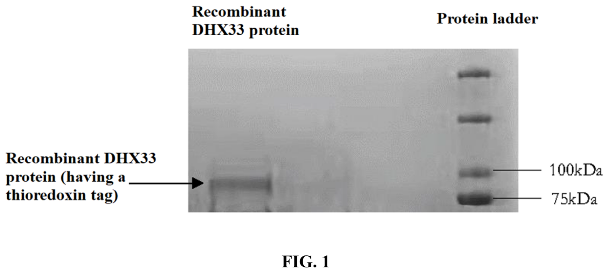

FIG. 1 shows the analysis of the results obtained after the SDS-PAGE separation and Coomassie brilliant blue staining of the recombinant DHX33 protein prepared by using the method of the present disclosure.

### DETAILED DESCRIPTION

In order to make the objects and technical solutions of the present disclosure more explicit, the embodiments of the present disclosure are described in detail below with reference to examples. However, those skilled in the art would understand that the following examples are merely for the illustration of the present disclosure and should not be construed as limiting the scope of the present disclosure.

The reagents or instruments used in the examples are all conventional products that are commercially available. If no specific conditions are specified, the routine conditions or the conditions suggested by the manufacturer shall be followed. The term "room temperature" used in the present disclosure refers to 20°C ± 5°C. When used for modifying certain numerical value or numerical range, the term "about" used in the present disclosure is intended to include this numerical value or numerical range as well as the error range (acceptable to those skilled in the art) of this numerical value or numerical range, for example, this error range is 10%, ±5%, ±4%, ±3%, ±2%, ±1%, ±0.5%, and the like.

The structures of the compounds described in the following examples are determined by nuclear magnetic resonance (NMR) and/or mass spectrometry (MS).

A Bruker 400 MHz NMR spectrometer is used as the measurement instrument of nuclear magnetic resonance (NMR), the solvents used for determination are deuterated methanol (CD₃OD), deuterated chloroform (CDCl₃) and hexadeuterated dimethyl sulfoxide (DMSO-d₆), and the internal standard substance is tetramethylsilane (TMS). In ¹H NMR, some of the hydrogen atoms may not give rise to peaks due to interference from a salt or a solvent.

The meanings of the abbreviations in the nuclear magnetic resonance (NMR) data in the following examples are as follows.

s: singlet, d: doublet, t: triplet, q: quartet, dd: doublet of doublets, qd: quartet of doublets, ddd: doublet of doublet of doublets, ddt: doublet of doublet of triplets, dddd: doublet of doublet of doublet of doublets, m: multiplet, br: broad singlet, J: coupling constant, Hz: Hertz, δ: chemical shift.

All chemical shift (δ) values are given in parts per million (ppm).

An Agilent 6120B mass spectrometer is used as the measurement instrument of mass spectrometry (MS), and the ion source is an electrospray ion source (ESI).

An Agilent 1200DAD high-pressure liquid chromatograph (chromatographic column: Sunfirc C18, 150 × 4.6 mm, 5 µm) and a Waters 2695-2996 high-pressure liquid chromatograph (chromatographic column: Gimini C18, 150 × 4.6 mm, 5 µm) are used for HPLC assay.

GF254 silica gel plates manufactured by Qingdao Marine Chemical Inc. are used as the silica gel plates for thin layer chromatography, the specification of the silica gel plates used for thin layer chromatography (TLC) is 0.15 mm to 0.2 mm, and the specification of the silica gel plates used for the separation and purification of the product via thin layer chromatography is 0.4 mm to 0.5 mm.

Silica gel (200 mesh to 300 mesh) manufactured by Qingdao Marine Chemical Inc. is generally used as the carrier in column chromatography.

Thin layer chromatography (TLC) is adopted to monitor the reaction progress in the examples. The solvent systems used in thin layer chromatography include (A) dichloromethane/methanol system and (B) petroleum ether/ethyl acetate system. The volume ratio of solvents is adjusted according to the polarity of the compound.

The eluent systems for column chromatography and the solvent systems for thin layer chromatography adopted for the purification of compounds include (A) dichloromethane/methanol system and (B) petroleum ether/ethyl acetate system. The volume ratio of solvents is adjusted according to the polarity of the compound, and a small amount of triethylamine or an acidic or basic reagent may also be added for adjustment.

### Synthesis of compounds

### Example 1 (Reference Example): Synthesis of Compound AB24333 ((E)-2-(3-(2-cyano-2-(6-methoxy-3H-imidazo[4,5-b]pyridin-2-yl)vinyl)-2,5-dimethyl-1H-pyrrol-1-yl)-5-methylthiophene-3-carbonitrile)

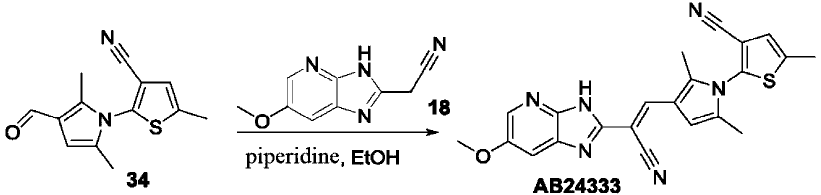

Compound **34** (2-(3-formyl-2,5-dimethyl-1H-pyrrol-1-yl)-5-methylthiophene-3-carbonitrile) (105 mg, 0.425 mmol, 1 eq) was dissolved in 2 ml of ethanol. Compound **18** (2-(6-methoxy-3H-imidazo[4,5-b]pyridin-2-yl)acetonitrile) (80 mg, 0.425 mmol, 1 eq) and two drops of piperidine were added thereto. The mixture was heated under reflux and stirred for 1 hour. After the completion of the reaction, the mixture was cooled to room temperature and filtered. The solid was collected and dried, so as to obtain Compound **AB24333** ((E)-2-(3-(2-cyano-2-(6-methoxy-3H-imidazo[4,5-b]pyridin-2-yl)vinyl)-2,5-dimethyl-1H-pyrrol-1-yl)-5-methylthiophene-3-carbonitrile) as a brown solid (150 mg, yield: 68.6%). MS (ESI) m/z: 415 [M+H+]. ¹H NMR (400 MHz, DMSO-d6) δ 13.15 (s, 1H), 8.16 (s, 1H), 8.06 (s, 1H), 7.55 (s, 1H), 7.33 (s, 1H), 6.98 (s, 1H), 3.84 (s, 3H), 2.53 (s, 3H), 2.31 (s, 3H), 2.10 (s, 3H).

### Example 2: Synthesis of Compound (E)-2-(3-(2-(1H-benzo[d]imidazol-2-yl)-2-cyanovinyl)-2,5-dimethyl-1H-pyrrol-1-yl)-5-methylthiophene-3-carbonitrile (AB24270)

Compound **AB24270** was obtained in the same manner as in Example 1 (yield: 82.3%), ¹H NMR (400 MHz, DMSO-d6) δ 12.83 (s, 1H), 8.13 (s, 1H), 7.55 (s, 2H), 7.33 (s, 1H), 7.19 (s, 2H), 6.98 (s, 1H), 2.47 (s, 3H), 2.31 (s, 3H), 2.09 (s, 3H).

### Example 3: Synthesis of Compound methyl (E)-2-(3-(2-(1H-benzo[d]imidazol-2-yl)-2-cyanovinyl)-2,5-dimethyl-1H-pyrrol-1-yl)-4,5-dimethylthiophene-3-carboxylate (AB24273)

Compound **AB24273** was obtained in the same manner as in Example 1 (yield: 65.7%), ¹H NMR (400 MHz, DMSO-d6) δ 12.77 (s, 1H), 8.09 (s, 1H), 7.51 (d, J = 1.6 Hz, 2H), 7.16 (s, 2H), 6.88 (s, 1H), 3.56 (s, 3H), 2.36 (s, 3H), 2.21 (d, J = 11.0 Hz, 6H), 1.97 (s, 3H).

### Example 4: Synthesis of Compound (E)-5-(3-(2-(1H-benzo[d]imidazol-2-yl)-2-cyanovinyl)-2,5-dimethyl-1H-pyrrol-1-yl)-3-methylthiophene-2,4-dicarbonitrile (AB24274)

Compound **AB24274** was obtained in the same manner as in Example 1 (yield: 76.9%), ¹H NMR (400 MHz, DMSO-d6) δ 12.87 (s, 1H), 8.14 (s, 1H), 7.55 (s, 2H), 7.21 (s, 2H), 7.01 (s, 1H), 2.48 (s, 3H), 2.35 (s, 3H), 2.15 (s, 3H).

### Example 5: Synthesis of Compound (E)-2-(3-(2-cyano-2-(6-methoxy-1H-benzo[d]imidazol-2-yl]vinyl)-2,5-dimethyl-1H-pyrrol-1-yl)benzonitrile (AB24307)

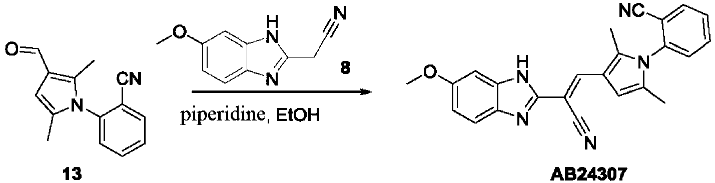

To a solution of Compound **8** (2-(6-methoxy-1H-benzo[d]imidazol-2-yl)acetonitrile) (84 mg, 0.45 mmol, 1.0 eq) in 2 mL of ethanol, Compound **13** (2-(3-formyl-2,5-dimethyl-1H-pyrrol-1-yl)benzonitrile) (100 mg, 0.45 mmol, 1.0 eq) and piperidine (39 mg, 0.45 mmol, 1.0 eq) were added. The mixture was heated under reflux and stirred for 1 hour. After the completion of the above operation, the mixture was cooled to room temperature and filtered. The solid was collected and dried, so as to obtain Compound **AB24307** (E)-2-(3-(2-cyano-2-(6-methoxy-1H-benzo[d]imidazol-2-yl]vinyl)-2,5-dimethyl-1H-pyrrol-1-yl)benzonitrile as a yellow solid (100 mg, yield: 47.6%). MS (ESI) m/z: 394 [M+H]⁺. ¹H NMR (DMSO-d6, 400 Hz): δ 12.68 (s, 1 H), 8.14 (s, 1 H), 8.09 (s, 1 H), 7.96 (s, 1 H), 7.78 (s, 1 H), 7.71 (s, 1 H), 7.45 (s, 1 H), 7.10-6.97 (m, 2H), 6.81 (s, 1 H), 3.78 (s, 3 H), 2.20 (s, 3 H), 1.99 (s, 3 H).

### Example 6: Synthesis of Compound methyl (E)-2-(3-(2-cyano-2-(6-methoxy-1H-benzo[ d]imidazol-2-yl)vinyl)-2,5-dimethyl-1H-pyrrol-1-yl)-4,5-dimethylthiophene-3-carboxylate (AB24322)

Compound **AB24322** was obtained in the same manner as in Example 5 (yield: 52.0%), ¹H NMR (DMSO-d6, 400 Hz): δ 12.58 (s, 1 H), 8.03 (s, 1 H), 7.42 (s, 1 H), 7.00 (s, 1 H), 6.87 (s, 1 H), 6.80 (s, 1 H), 3.77 (s, 3 H), 3.57 (s, 3 H), 2.38 (s, 3 H), 2.22 (d, J = 14.8 Hz, 6 H), 1.98 (s, 3 H).

### Example 7: Synthesis of Compound (E)-2-(1H-benzo[d]imidazol-2-yl)-3-(3,5-dimethyl-1-(3-(trifluoromethyl)phenyl)-1H-pyrazol-4-yl)acrylonitrile (AB24285)

### (1) Synthesis of Compound 16 (3,5-dimethyl-1-(3-(trifluoromethyl)phenyl)-1H-pyrazole)

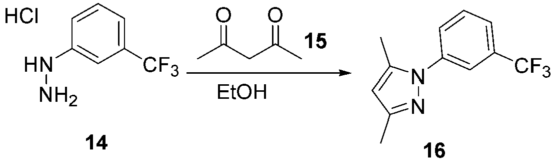

Compound **14** (3-(trifluoromethyl)phenylhydrazine hydrochloride) (1.0 g, 4.7 mmol, 1.0 eq) was dissolved in ethanol (15 mL), and Compound **15** (acetylacetone) (471 mg, 4.7 mmol, 1.0 eq) was added thereto. The mixture was heated under reflux for 3 hours. After the mixture was concentrated, the residue was dissolved in water (20 mL) and extracted three times with ethyl acetate (20 mL × 3). The organic layers were dried, filtered and concentrated. The residue was purified by flash column chromatography (petroleum ether/ethyl acetate = 20/1), so as to obtain Compound **16** (3,5-dimethyl-1-(3-(trifluoromethyl)phenyl)-1H-pyrazole) as a yellow solid powder (900 mg, yield: 79.7%). MS (ESI) m/z: 241 [M+H]⁺. TLC: petroleum ether/ethyl acetate (3:1); R_{f} (Compound 14) = 0.3; R_{f} (Compound 16) = 0.6.

### (2) Synthesis of Compound 17 (3,5-dimethyl-1-(3-(trifluoromethyl)phenyl)-1H-pyrazole-4-carbaldehyde)

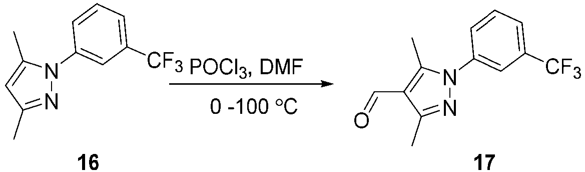

Phosphorus oxychloride (400 mg, 2.63 mmol, 0.7 eq) was added to dimethylformamide (10 mL, 0°C) in the presence of nitrogen gas. The mixture was stirred at 0°C for 30 minutes and then heated to room temperature. A solution of Compound **16** (3,5-dimethyl-1-(3-(trifluoromethyl)phenyl)-1H-pyrazole) (900 mg, 3.75 mmol, 1.0 eq) in 10 mL of dimethylformamide was added to the above-mentioned reaction system. The mixture was heated to 100°C, and was stirred for 1 hour in the presence of nitrogen gas. After the reaction mixture was cooled, all the reaction products were poured into ice water, and the pH of the resulting mixture was adjusted to 10 with 30% sodium hydroxide solution. The mixture was extracted with ethyl acetate and then washed with brine. The organic layer was dried over sodium sulfate, filtered and concentrated. The residue was purified by flash column chromatography (petroleum ether/ethyl acetate = 50/1 to 10/1), so as to obtain Compound **17** (3,5-dimethyl-1-(3- (trifluoromethyl)phenyl)-1H-pyrazole-4-carbaldehyde) as a yellow solid powder (320 mg, yield: 31.8%). MS (ESI) m/z: 269 [M+H]⁺. TLC: petroleum ether/ethyl acetate (3:1); R_{f} (Compound 16) = 0.6; R_{f} (Compound 17) = 0.4.

### (3) Synthesis of Compound AB24285 ((E)-2-(1H-benzo[d]imidazol-2-yl)-3-(3,5-dimethyl-1-(3-(trifluoromethyl)phenyl)-1H-pyrazol-4-yl)acrylonitrile)

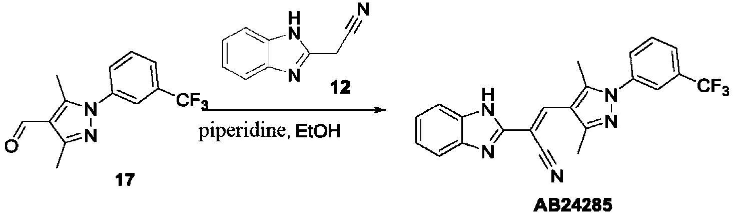

Compound **17** (3,5-dimethyl-1-(3-(trifluoromethyl)phenyl)-1H-pyrazole-4-carbaldehyde) (100 mg, 0.37 mmol, 1.0 eq) was dissolved in ethanol (2.0 mL). Compound **12** (2-cyanomethylbenzimidazole) (59 mg, 0.37 mmol, 1.0 eq) and piperidine (32 mg, 0.371 mmol, 1.0 eq) were added thereto. The mixture was heated under reflux and stirred for 1 hour. After the completion of the reaction, the resulting mixture was cooled to room temperature, filtered and concentrated. The solid was collected and dried so as to obtain **AB24285** ((E)-2-(1H-benzo[d]imidazol-2-yl)-3-(3,5-dimethyl-1-(3-(trifluoromethyl)phenyl)-1H-pyrazol-4-yl)acrylonitrile) as a yellow solid powder (40 mg, yield: 26.3%). MS (ESI) m/z: 408 [M+H]⁺. ¹H NMR (400 MHz, DMSO) δ 8.11 (s, 1H), 7.80 (s, 3H), 7.75 - 7.61 (m, 5H), 7.55-7.40 (m, 4H), 7.22 (s, 4H), 2.37 (s, 6H), 2.01 (s, 1H), 1.80 (s, 1H).

### Example 8: Synthesis of Compound (E)-2-(1H-benzo[d]imidazol-2-yl)-3-(3,5-dimethyl-1-phenyl-1H-pyrazol-4-yl)acrylonitrile (AB24284)

**AB24284** was obtained in the same manner as in Example 7 (yield: 50%), ¹H NMR (400 MHz, CDCl3) δ 9.59 (s, 1H), 8.45 (s, 1H), 7.78 (s, 1H), 7.58 - 7.39 (m, 6H), 7.32 (d, J = 6.6 Hz, 2H), 2.48 (s, 6H).

### Example 9: Synthesis of Compound (E)-2-(1H-benzo[d]imidazol-2-yl)-3-(3,5-dimethyl-1-(2-(trifluoromethoxy)phenyl)-1H-pyrazol-4-yl)acrylonitrile (AB24290)

**AB24290** was obtained in the same manner as in Example 7 (yield: 20%), ¹H NMR (400 MHz, dmso) δ 8.21 (s, 2H), 7.82 (s, 1H), 7.75 - 7.35 (m, 20H), 7.21-7.19 (m, 8H), 7.05 (s, 1H), 6.92 (s, 1H), 2.37 (s, 6H), 2.00 (s, 3H), 1.79 (s, 3H).

### Example 10: Synthesis of Compound AB24331 ((E)-2-(3-(2-cyano-2-(6-(trifluoromethyl)-1H-benzo[d]imidazol-2-yl)vinyl)-2,5-dimethyl-1H-pyrrol-1-yl)-4,5-dimethylthiophene-3-carbonitrile)

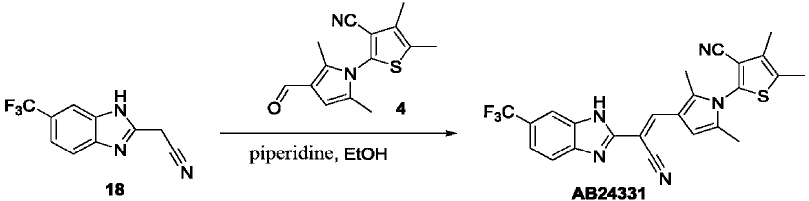

Compound **18** (2-(6-(trifluoromethyl)-1H-benzo[d]imidazol-2-yl)acetonitrile) (50 mg, 0.222 mmol, 1 eq) was dissolved in 2 mL of ethanol. Compound **4** (2-(3-formyl-2,5-dimethyl-1H-pyrrol-1-yl)-4,5-dimethylthiophene-3-carbonitrile) (68 mg, 0.222 mmol, 1 eq) and two drops of piperidine were added thereto. The mixture was heated under reflux and stirred for 2 hours. After the completion of the reaction, the mixture was cooled to room temperature and filtered. The yellow solid was collected and dried, so as to obtain **AB24331** ((E)-2-(3-(2-cyano-2-(6-(trifluoromethyl)-1H-benzo[d]imidazol-2-yl)vinyl)-2,5-dimethyl-1H-pyrrol-1-yl)-4,5-dimethylthiophene-3-carbonitrile) (74 mg, yield: 71.8%). MS (ESI) m/z: 466.25 [M+H+]. ¹H NMR (400 MHz, DMSO-*d*₆) δ 8.21 (s, 1H), 7.90 (s, 1H), 7.73-7.71 (m, 1H), 7.50 (d, *J =* 8.4 Hz, 1H), 6.98 (s, 1H), 2.42 (s, 3H), 2.32 (s, 3H), 2.24 (s, 3H), 2.10 (s, 3H).

### Example 11: Synthesis of Compound (E)-2-(3-(2-cyano-2-(6-(trifluoromethoxy)-1H-benzo[d]imidazol-2-yl)vinyl)-2,5-dimethyl-1H-pyrrol-1-yl)-4,5-dimethylthiophene-3-carbonitrile (AB24339)

**AB24339** was obtained in the same manner as in Example 10 (yield: 73.7%). **¹H NMR (400 MHz, DMSO-*d*₆)** δ 8.16 (s, 1H), 7.61-7.55 (m, 2H), 7.18 (d, *J =* 7.6 Hz, 1H), 6.97 (s, 1H), 2.42 (s, 3H), 2.28 (d, *J =* 25.2 Hz, 6H), 2.10 (s, 3H).

### Example 12: Synthesis of Compound AB24343 ((Z)-2-(3-(2-cyano-2-(5-methoxy-1H-indol-2-yl)vinyl)-2,5-dimethyl-1H-pyrrol-1-yl)-5-methylthiophene-3-carbonitrile)

### (1) Synthesis of Compound 21 ((5-methoxy-1H-indol-2-yl)methanol)

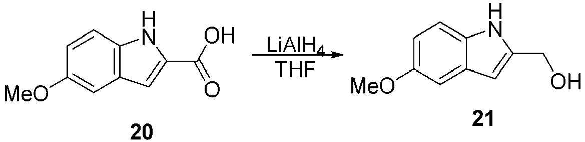

Compound **20** (5-methoxyindole-2-carboxylic acid) (2 g, 10.5 mmol, 1 eq) was dissolved in anhydrous tetrahydrofuran THF (20 mL). Lithium aluminum hydride (0.438 g, 11.05 mmol, 1.05 eq) was slowly added at 0°C. The mixture was stirred at room temperature for 5 hours. The reaction mixture was cooled to 0°C, 5 mL of NaOH solution was added thereto, and the resulting mixture was stirred at 25°C for 5 min. The mixture was extracted with ethyl acetate, dried over anhydrous sodium sulfate and concentrated. The residue was purified by flash column chromatography (petroleum ether/ethyl acetate = 5:1 to 2:1), so as to obtain Compound **21** ((5-methoxy-1H-indol-2-yl)methanol) (956 mg, yield: 51.4%). TLC: petroleum ether/ethyl acetate (1/1); R_{f} (*Compound 20*) = 0.3; R_{f} (*Compound 21*) = 0.6; ¹H NMR (400 MHz, CDCl₃) *δ* 8.26 (s, 1H), 7.30-7.16 (m, 1H), 7.03 (s, 1H), 6.84 (d, *J =* 8.9 Hz, 1H), 6.33 (s, 1H), 4.79 (s, 2H), 3.84 (s, 3H).

### (2) Synthesis of Compound 22 (methyl (5-methoxy-1H-indol-2-yl)benzoate)

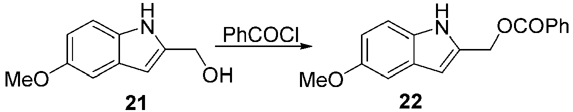

Compound **21** ((5-methoxy-1H-indol-2-yl)methanol) (356 mg, 2.03 mmol, 1.0 eq) and triethylamine TEA (410 mg, 4.06 mmol, 2 eq) were dissolved in tetrahydrofuran (5 mL), and benzoyl chloride (430 g, 3.05 mmol, 1.5 eq) was added thereto. The mixture was stirred and reacted at room temperature for 10 minutes. Afterwards, the reaction was quenched with sodium bicarbonate hydrate, and the resulting mixture was extracted with ethyl acetate. The organic layer was washed with brine, dried over anhydrous sodium sulfate and filtered. The filtrate was concentrated to obtain Compound **22** (methyl (5-methoxy-1H-indol-2-yl)benzoate) (478 mg, yield: 83.7%), which was directly used in the next step without the need of further purification. TLC: petroleum ether/ethyl acetate (3/1); R_{f} (*Compound 21*) = 0.2; R_{f} (*Compound* 22) = 0.7; ¹H NMR (400 MHz, DMSO-d6) δ 11.11 (s, 1H), 7.95 (d, *J =* 7.7 Hz, 2H), 7.61 (t, *J* = 7.6 Hz, 1H), 7.48 (t, *J =* 7.7 Hz, 2H), 7.22 (d, *J =* 8.8 Hz, 1H), 6.97 (s, 1H), 6.70 (d, *J =* 8.7 Hz, 1H), 6.42 (s, 1H), 5.38 (s, 2H), 3.68 (s, 3H).

### (3) Synthesis of Compound 23 (2-(5-methoxy-1H-indol-2-yl)acetonitrile)

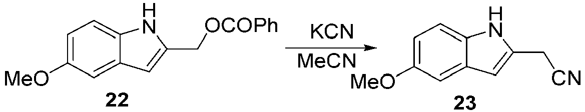

Compound **22** (methyl (5-methoxy-1H-indol-2-yl)benzoate) (250 mg, 0.89 mmol, 1.0 eq) was dissolved in acetonitrile (3 mL), and potassium cyanide (116 mg, 1.78 mmol, 2.0 eq) was added thereto. The mixture was stirred at 80°C for 24 hours. After the completion of the reaction, the resulting mixture was cooled to room temperature, and saturated aqueous sodium bicarbonate solution was added thereto. The mixture was extracted with ethyl acetate, and the organic layer was dried over anhydrous sodium sulfate and concentrated. The residue was purified by flash column chromatography (petroleum ether/ethyl acetate = 3:1 to 1:1), so as to obtain Compound **23** (2-(5-methoxy-1H-indol-2-yl)acetonitrile) (30 mg, yield: 18.1%). TLC: petroleum ether/ethyl acetate (3/1); R_{f} (*Compound 22*) = 0.25; R_{f} (*Compound 23*) = 0.5; ¹H NMR (400 MHz, CDCl₃) *δ* 8.12 (s, 1H), 7.41-7.12 (m, 1H), 7.03 (s, 1H), 6.87 (d, *J =* 8.5 Hz, 1H), 6.41 (s, 1H), 3.90 (s, 2H), 3.85 (s, 3H).

### (4) Synthesis of Compound AB24343 ((Z)-2-(3-(2-cyano-2-(5-methoxy-1H-indol-2-yl)vinyl)-2,5-dimethyl-1H-pyrrol-1-yl)-5-methylthiophene-3-carbonitrile)

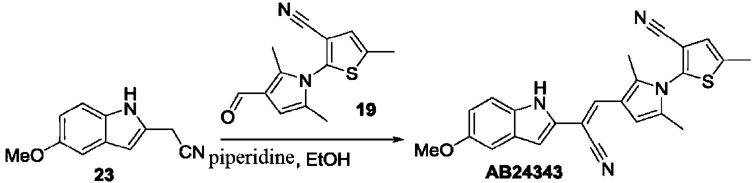

Compound **23** (2-(5-methoxy-1H-indol-2-yl)acetonitrile) (30 mg, 0.16 mmol, 1 eq) was dissolved in ethanol (1 mL). Compound **19** (2-(3-formyl-2,5-dimethyl-1H-pyrrol-1-yl)-5-methylthiophene-3-carbonitrile) (39 mg, 0.16 mmol, 1 eq) and two drops of piperidine were added thereto. The mixture was heated under reflux and stirred for 2 hours. After the completion of the reaction, the resulting mixture was cooled to room temperature and filtered. The solid was collected and dried, so as to obtain Compound **AB24343** ((Z)-2-(3-(2-cyano-2-(5-methoxy-1H-indol-2-yl)vinyl)-2,5-dimethyl-1H-pyrrol-1-yl)-5-methylthiophene-3-carbonitrile) as a yellow solid (20 mg, yield: 30.3%). MS (ESI) m/z: 413.05 [M+H⁺]. ¹H NMR (400 MHz, DMSO-*d*₆) *δ* 11.41 (s, 1H), 7.68 (s, 1H), 7.29 (s, 1H), 7.21 (d, *J =* 8.6 Hz, 1H), 6.99 (s, 1H), 6.86 (s, 1H), 6.73 (d, *J =* 8.4 Hz, 1H), 6.49 (s, 1H), 3.70 (s, 3H), 2.49 (s, 3H), 2.24 (s, 3H), 2.06 (s, 3H).

### Example 13: Synthesis of Compound AB24264 ((E)-2-(3-(2-(1-(1-H-benzo[d]imidazol-2-yl]-2-cyanovinyl])-2,5-dimethyl-1H-pyrrol-1-yl)-4,5-dimethylthiophene-3-carbonitrile)

### (1) Synthesis of Compound 3 (2-(2,5-dimethyl-1H-pyrrol-1-yl)-4,5-dimethylthiophene-3-carbonitrile)

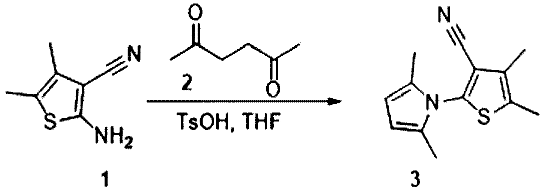

Compound **1** (2-amino-3-cyano-4,5-dimethylthiophene) (1.0 g, 6.57 mmol, 1.0 eq) was dissolved in tetrahydrofuran (30 mL). Compound **2** (2,5-hexanedione) (1.2 g, 10.51 mmol, 1.6 eq), 3A molecular sieve (2 g) and p-toluenesulfonic acid hydrate (450 mg, 2.63 mmol, 0.4 eq) were added thereto. The reactants were heated under reflux and stirred overnight. The solid was filtered off, and the filtrate was concentrated. The residue was purified by flash column chromatography (petroleum ether /ethyl acetate = 100/1), so as to obtain Compound **3** (2-(2,5-dimethyl-1H-pyrrol-1-yl)-4,5-dimethylthiophene-3-carbonitrile) as a yellow solid (650 mg, yield: 42.9%). MS (ESI) m/z: 231 [M+H]⁺. **TLC**: petroleum ether/ethyl acetate (10: 1); **R_{f}** (Compound ***1***) = 0.2; **R_{f}** (Compound ***3***) = 0.7.

### (2) Synthesis of Compound 4 (2-(3-formyl-2,5-dimethyl-1H-pyrrol-1-yl)-4,5-dimethylfuran-3-carbonitrile)

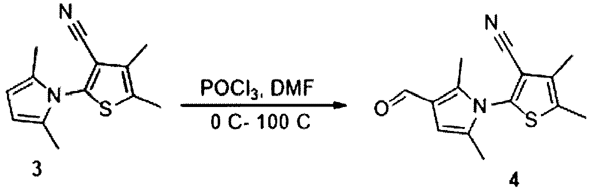

Under the protection of nitrogen gas, phosphorus oxychloride (432 mg, 2.82 mmol, 1.0 eq) was added dropwise to dimethylformamide (5 mL) at 0°C. The mixture was stirred at 0°C for 30 minutes and was then warmed to room temperature. A solution of Compound **3** (2-(2,5-dimethyl-1H-pyrrol-1-yl)-4,5-dimethylthiophene-3-carbonitrile) (650 mg, 2.82 mmol, 1.0 eq) in 2 ml of dimethylformamide was added to the above-mentioned reaction system. The mixture was heated to 100°C, and was stirred for 2 hours under the protection of nitrogen gas. After the reaction mixture was cooled, the reaction mixture was poured into ice water, and then the pH of the resulting mixture was adjusted to 10 with 30% NaOH solution. The reaction solution was extracted with ethyl acetate and washed with brine. Afterwards, the organic layer was dried over anhydrous sodium sulfate, filtered and concentrated. The residue was purified by flash column chromatography (petroleum ether/ethyl acetate = 100/1), so as to obtain Compound **4** (2-(3-formyl-2,5-dimethyl-1H-pyrrol-1-yl)-4,5-dimethylfuran-3-carbonitrile) as a yellow solid (390 mg, yield: 52.5%). MS (ESI) m/z: 259 [M+H]⁺. **TLC:** petroleum ether/ethyl acetate (20: 1); **R_{f}** (***Compound 3***) = 0.7; **R_{f}** (***Compound 4***) = 0.5.

### (3) Preparation of Compound AB24264 ((E)-2-(3-(2-(1-(1-H-benzo[d]imidazol-2-yl] -2-cyanovinyl])-2,5-dimethyl-1H-pyrrol-1-yl)-4,5-dimethylthiophene-3-carbonitrile)

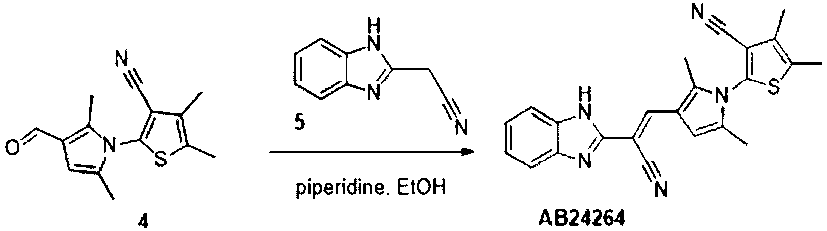

Compound **4** (2-(3-formyl-2,5-dimethyl-1H-pyrrol-1-yl)-4,5-dimethylfuran-3-carbonitrile) (160 mg, 0.62 mmol, 1.0 eq) was dissolved in ethanol (3 mL). Compound 5 (2-cyanomethylbenzimidazole) (98 mg, 0.62 mmol, 1.0 eq) and piperidine (53 mg, 0.62 mmol, 1.0 eq) were added thereto. The mixture was heated under reflux and stirred for 1 hour. After the completion of the reaction, the mixture was cooled to room temperature and filtered. The solid was collected and dried, so as to obtain Compound **AB24264** ((E)-2-(3-(2-(1-(1-H-benzo[d]imidazol-2-yl]-2-cyanovinyl])-2,5-dimethyl-1H-pyrrol-1-yl)-4,5-dimethylthiophene-3-carbonitrile) as a yellow solid (120 mg, yield: 48.8%). MS (ESI) m/z: 398 [M+H]⁺. **HNMR** ¹H NMR (400 MHz, CDCl₃) δ 8.12 (s, 1H), 7.53 (s, 2H), 7.18 (d, *J =* 3.1 Hz, 2H), 6.96 (s, 1H), 2.41 (s, 3H), 2.30 (s, 3H), 2.23 (s, 3H), 2.09 (s, 3H).

### Example 14: Synthesis of Compound AB24354 ((E)-5-(3-(2-cyano-2-(5-methoxy-1H-benzo[d]imidazol-2-yl)vinyl)-2,5-dimethyl-1H-pyrrol-1-yl)-2-methyloxazole-4-carbonitrile)

### (1) Synthesis of Compound 3 (5-(2,5-dimethyl-1H-pyrrol-1-yl)-2-methyloxazole-4-carbonitrile)

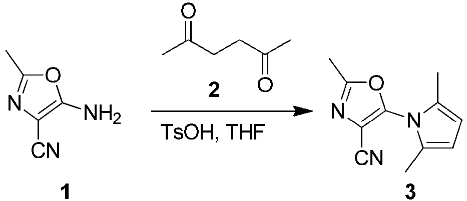

Compound **1** (5-amino-2-methyloxazole-4-carbonitrile) (2 g, 16.26 mmol, 1.0 eq) was dissolved in tetrahydrofuran (20 mL). Afterwards, Compound **2** (2,5-hexanedione) (2.78 g, 24.39 mmol, 1.5 eq), 3A molecular sieve 3A MS (2.0 g) and p-toluenesulfonic acid hydrate (300 mg, 3.252 mmol, 0.2 eq) were added thereto. The mixture was heated under reflux and stirred overnight. The solid was collected, filtered and concentrated. The residue was purified by flash column chromatography (petroleum ether/ethyl acetate = 20/1), so as to obtain Compound **3** (5-(2,5-dimethyl-1H-pyrrol-1-yl)-2-methyloxazole-4-carbonitrile) as a white solid (2.3 g, yield: 70.3%). MS (ESI) m/z: 202 [M+H]⁺. TLC: petroleum ether/ethyl acetate (10: 1); R_{f} (*Compound 1*) = 0.2; R_{f} (*Compound 3*) = 0.7.

### (2) Synthesis of Compound 4 (5-(3-formyl-2,5-dimethyl-1H-pyrrol-1-yl)-2-methyloxazole-4-carbonitrile)

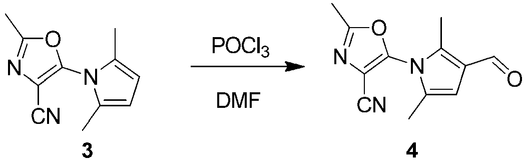

Under the protection of nitrogen gas, phosphorus oxychloride (453 mg, 2.98 mmol, 1.2 eq) was added dropwise to dimethylformamide (30 mL) at zero degrees Celsius. The mixture was stirred at zero degrees Celsius for 30 minutes and then warmed to room temperature. A solution of Compound **3** (5-(2,5-dimethyl-1H-pyrrol-1-yl)-2-methyloxazole-4-carbonitrile) (500 mg, 2.48 mmol, 1.0 eq) in 5 mL of dimethylformamide was added to the above-mentioned reaction system. Under the protection of nitrogen gas, the above-mentioned mixture was heated to 100 degrees Celsius and stirred for two hours. After the reaction mixture was cooled, the mixture was poured into ice water, and the pH of the resulting mixture was adjusted to 10 with 30% sodium hydroxide solution. The mixture was extracted with ethyl acetate and washed with brine. The organic layer was dried over anhydrous sodium sulfate, filtered and concentrated. The residue was purified by flash column chromatography (petroleum ether/ethyl acetate = 100/1), so as to obtain Compound **4** (5-(3-formyl-2,5-dimethyl-1H-pyrrol-1-yl)-2-methyloxazole-4-carbonitrile) as a yellow solid (300 mg, yield: 52 %). MS (ESI) m/z: 230 [M+H]⁺. TLC: petroleum ether/ethyl acetate (5: 1); R_{f} (*Compound 3*) = 0.7; R_{f} (*Compound 4*) = 0.3.

### (3) Synthesis of Compound AB24354 ((E)-5-(3-(2-cyano-2-(5-methoxy-1H-benzo[d]imidazol-2-yl)vinyl)-2,5-dimethyl-1H-pyrrol-1-yl)-2-methyloxazole-4-carbonitrile)

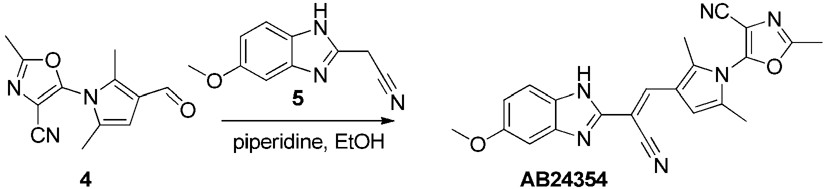

Compound **4** (5-(3-formyl-2,5-dimethyl-1H-pyrrol-1-yl)-2-methyloxazole-4-carbonitrile) (52 mg, 0.228 mmol, 1 eq) was dissolved in 1 mL of ethanol. Compound **5** (2-(5-methoxy-1H-benzo[d]imidazol-2-yl)acetonitrile) (43 mg, 0.228 mmol, 1 eq) and two drops of piperidine were added thereto. The mixture was heated under reflux and stirred for 2 hours. After the completion of the reaction, the mixture was cooled to room temperature and filtered. The solid was collected and dried, so as to obtain Compound **AB24354** ((E)-5-(3-(2-cyano-2-(5-methoxy-1H-benzo[d]imidazol-2-yl)vinyl)-2,5-dimethyl-1H-pyrrol-1-yl)-2-methyloxazole-4-carbonitrile) as a yellow solid (30 mg, yield: 33.2%). MS (ESI) m/z: 399 [M+H+]. ¹H NMR (400 MHz, DMSO-*d*₆) δ 12.76 (s, 1H), 8.50 (s, 1H), 7.45 (s, 1H), 6.98 (s, 2H), 6.82 (d, *J =* 8.8 Hz, 1H), 3.77 (s, 3H), 2.54 (s, 3H), 2.39 (s, 3H), 2.20 (s, 3H).

### Pharmacological activity test and analysis of results

### Preparation of recombinant DHX33

Regarding the isolation and purification of proteins, please refer to Wang X, Ge W, and Zhang Y. Recombinant DHX33 Protein Possesses Dual DNA/RNA Helicase Activity. Biochemistry. 2019;58 (4):250-8. RNA helicase gene (mouse DHX33 gene) was cloned into a pET32M-3C vector (between BamH I/Not I restriction sites). Afterward, the plasmid was transformed into BL-21pLysS (DE3) E. coli. 0.5 mM isopropyl 1-thio-β-D-galactopyranoside (IPTG) was added, and the expression of the recombinant protein was induced at 16°C for 16 hours. Cells were allowed to precipitate and were resuspended in a cell lysis buffer [50 mM Tris-HCl (pH7.2), 150 mM NaCl, 1%Triton X-100, and 50 mM imidazole supplemented with a protease inhibitor]. Thereafter, cells were subjected to ultrasonic treatment and centrifuged at a rotation speed of 13000 rpm for 25 minutes. The supernatant was incubated with the nickel-nitrilotriacetic acid beads equilibrated in Tris buffer, followed by sufficient washing. Thereafter, the purified protein was eluted with a solution of 300 mM imidazole in Tris buffer, and was then subjected to dialysis against an imidazole-free Tris buffer at 4°C overnight.

Fig. 1 showed the analysis of the results obtained after the recombinant DHX33 protein prepared by the above-mentioned method was separated by SDS-PAGE and then stained with Coomassie brilliant blue. The arrow in the figure indicated the target recombinant DHX33 protein (containing a thioredoxin tag), and the molecular weight of the target band was 90 kDa.

### Analysis of the helicase activity of DHX33

The components involved in the reaction for testing the helicase activity were added to a 96-well opaque white plate. The method was outlined as follows. The 96-well plate was coated with neutravidin at a final concentration of 10 µg/mL (100 µL/well) overnight at 4°C. Afterwards, the neutravidin-coated plate was blocked with 100 µL of 0.1% (w/v) BSA (dissolved in normal PBS) at 22°C for 2 hours. After washing, a DNA duplex (2.5 ng) that was resulted from the annealing of two single-stranded DNA oligonucleotides (the sequence of one single strand was biotin-labeled 5'-GCTGACCCTGCTCCCAATCGTAATCTATAG-3'; and the sequence of the other single strand was DIG-labeled 5'-CGATTGGGAGCAGGGTCAGC-3') [the annealing reaction was carried out in PBS (pH 7.0) containing 1M NaCl] was added, and the resulting mixture was incubated at 22°C for 4 hours. After the addition of 90 µL of the reaction mixture, the helicase reaction was initiated [0.25 µg of purified full-length DHX33 protein, dissolved in 25 mM 4-MOPS (pH 7.0), 5 mM ATP, 2 mM DTT, 3 mM MnCl₂ and 100 µg/mL BSA]. The reaction was conducted at 37°C for 60 minutes. After being washed, each well was incubated with a blocking solution [10% (w/v) BSA in 0.1 M maleic acid and 0.15 M NaCl (pH 7.5)] for 30 minutes, and was then incubated with 20 µL of antibody solution (anti-DIG-AP, Roche, in blocking buffer) for 30 minutes. After being washed with 100 µL of detection buffer [0.1 M Tris-HCl and 0.1 M NaCl (pH 9.5)], each well was added with 1 µL of chemiluminescent substrate (CSPD-0.25 mM), and the plate was incubated at 17°C for 5 minutes. Afterwards, the plate was patted dry and incubated at 37°C for 30 minutes. The remaining DIG-AP marker control in each well was counted for 10 minutes by a luminescence multi-well plate reader (Enspire, PerkinElmer). Higher reading indicated weaker helicase activity.

Positive control and negative controls were set for this acticity assay. Among them, one negative control group, which differed from the experimental group only in that the former was not added with ATP (other conditions were the same as the experimental group), was used for the comparison with the experimental group; another negative control group, which differed from the experimental group only in that the former was not added with DHX33 protein (other conditions were the same as the experimental group), was used for the comparison with the experimental group. The positive control group was set for the comparison with the wild-type DHX33 protein (standard substance). The results obtained from the comparison with DHX33 protein (standard substance) indicated that DHX33 protein prepared by the above-mentioned method had helicase activity.

Regarding the specific analytical method of helicase activity, please refer to Wang X, Ge W, and Zhang Y. Recombinant DHX33 Protein Possesses Dual DNA/RNA Helicase Activity. Biochemistry. 2019;58 (4):250-8.

### Quantitative real-time PCR

Primers were designed by online "Realtime PCR Tool"of IDT (http://sg.idtdna.com/site), purchased from BGI (Shenzhen) Corporation. Total RNA was extracted by using High Pure RNA Isolation Kit (Roche), and was then transcribed into cDNA by using PrimeScript Mix Kit (Takara). Real-time PCR was conducted by using ABI One step plus cycler, and was managed by using the corresponding software. In order to analyze mRNA level, SYBR green Supermix (Bio-Rad) was used, and after the data were normalized to GAPDH value, ^{△△}CT value was used to calculate and quantify the transcript. The melting curve was used for the confirmation of the amplification of a single product. Regarding the sequence of the primers, please refer to Yuan B, Wang X, Fan C, You J, Liu Y, Weber JD, Zhong H, and Zhang Y. DHX33 Transcriptionally Controls Genes Involved in the Cell Cycle. Molecular and cellular biology. 2016;36 (23):2903-17.

### Regulation of known downstream genes of DHX33 by compounds

U251 cells were treated with AB24254 (having a concentration of 30nM, 40nM and 50nM, respectively) or an equal volume of dimethyl sulfoxide for 16 hours. Total RNA was extracted and was then subjected to reverse transcription to obtain cDNA. Thereafter, real-time quantitative PCR was conducted to analyze the transcript levels of the target genes (including CCNE2, E2F1 and MCM3) in each sample, and GAPDH was used as the internal reference.

### Inhibition of the helicase activity of DHX33 by compounds

Compounds having a series of concentration (the concentrations were set at 1 nM, 5 nM, 10 nM, 20 nM, 50 nM, 100 nM, 250 nM, 500 nM, and 1000 nM) were used to conduct the helicase activity assay of DHX33 protein *in vitro.* The half-maximal inhibitory concentrations of the compounds of the present disclosure on the helicase activity of DHX33 protein were as shown in Table 1. As could be seen from Table 1, the compounds of the present disclosure had significant inhibitory effects on the helicase activity of DHX33 protein.

**Table 1: Assay of the inhibitory effects of the compounds on the helicase activity of DHX33 protein**

| Compound | Structure | IC₅₀ | Compound | Structure | IC₅₀ |
|---|---|---|---|---|---|
| Compound as control | 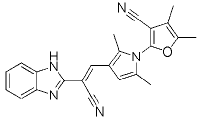 | * | | | |
| AB24264 | 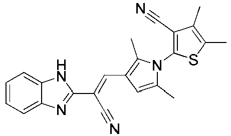 | ** | AB24270 | 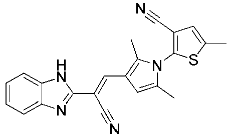 | *** |
| AB24259 | 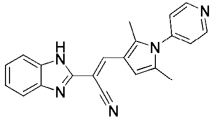 | ** | AB24322 | 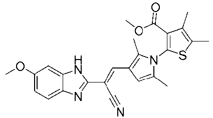 | ** |
| AB24321 | 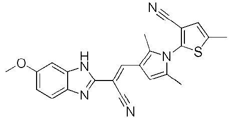 | **** | | | |

| | | | | | |
|---|---|---|---|---|---|
| * denoted that the half-maximal inhibitory concentration was 400 nM or more and less than 1000 nM; ** denoted that the half-maximal inhibitory concentration was 100 nM or more and less than 400 nM; *** denoted that the half-maximal inhibitory concentration was 20 nM or more and less than 100 nM; and **** denoted that the half-maximal inhibitory concentration was less than 20 nM. | | | | | |

### Cell culture and sources

U251-MG cells were purchased from the cell bank of the Chinese Academy of Sciences. Cells were cultured in MEM medium containing 10% fetal bovine serum (FBS), 2 mM L-glutamine, streptomycin and penicillin. The growth conditions were set as follows: a humidified cell incubator (37°C, 5% CO₂). Cells were passaged every 3 days and were discarded after 10 passages.

### Determination of half-maximal cell-inhibitory concentrations (IC₅₀)

U251-MG cells (a DHX33-overexpressing cancer cell strain) were seeded on a 96-well plate at 1×10⁴ cells/100µl/well. After the cells were completely attached, the compounds were added to the cell culture medium at a concentration of 5 nM, 10 nM, 25 nM, 50 nM, 100 nM, 250 nM, 500 nM, 1000 nM, 2000 nM, 5000 nM, 10 µM or 20 µM, and the resulting mixture was uniformly mixed with a multichannel pipette. After the cells were incubated with the compound for 48 hours, CCK-8 reagent (Yeasen Biotechnology (Shanghai) Co., Ltd.) was added to the medium in the 96-well plate in accordance with the standard procedure. After being incubated for 2 hours, the plate was read with a microplate reader (OD = 450nm). The experiment was repeated three times, the inhibiton curve of the compound at different concentrations was plotted, and the half-maximal inhibitory concentration (IC₅₀) of the compound was calculated.

**Table 2: Determination of the half-maximal inhibitory concentrations of compounds on U251-MG cells**

| Compound No. | Structure of compound | IC₅₀ | Compound No. | Structure of compound | IC₅₀ |
|---|---|---|---|---|---|
| Compound as control | 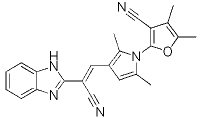 | * | | | |
| | | | AB24237 | 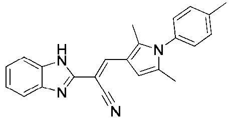 | * |
| AB24321 | 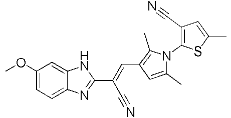 | *** | AB24223 | 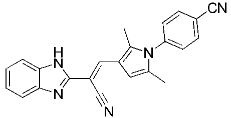 | * |
| AB24267 | 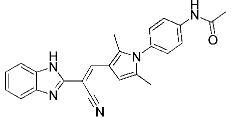 | * | AB24227 | 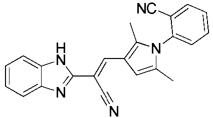 | * |
| AB24233 | 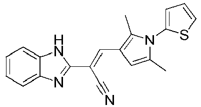 | * | AB24258 | 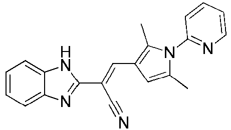 | * |
| AB24259 | 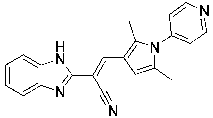 | ** | AB24264 | 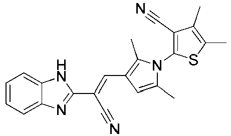 | ** |
| AB24270 | 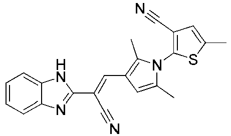 | *** | AB24273 | 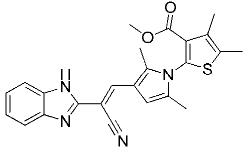 | *** |
| AB24274 | 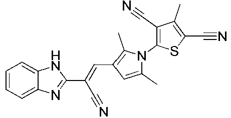 | * | AB24276 | 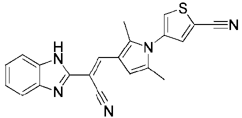 | * |
| AB24277 | 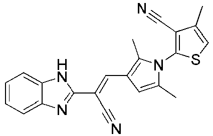 | * | AB24322 | 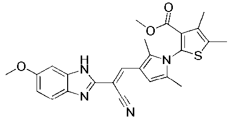 | ** |
| AB24284 | 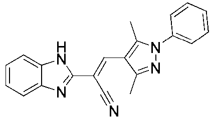 | * | AB24285 | 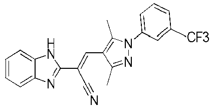 | * |
| AB24307 | 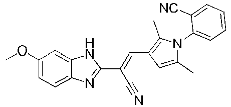 | * | AB24290 | 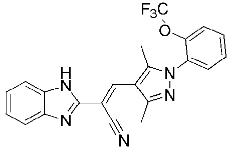 | * |

| | | | | | |
|---|---|---|---|---|---|
| * denoted that the half-maximal inhibitory concentration was 5 µM or more and less than 20 µM; ** denoted that the half-maximal inhibitory concentration was 1 µM or more and less than 5 µM ; *** denoted that the half-maximal inhibitory concentration was 100 nM or more and less than 1000 nM; **** denoted that the half-maximal inhibitory concentration was less than 100 nM. | | | | | |

As could be seen from Table 2, the compounds of the present disclosure had significant inhibitory effects on U251-MG cells (a DHX33-overexpressing cancer cell strain).

## Claims

1. A compound having the structure of formula II or a pharmaceutically acceptable form thereof: 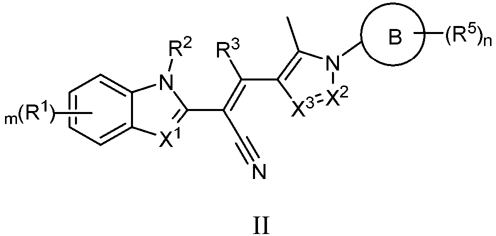 wherein
each R¹ is independently halogen, amino, -NH(C₁₋₄ alkyl), -N(C₁₋₄ alkyl)₂, nitro, C₁₋₄ alkyl, C₁₋₄ haloalkyl, C₁₋₄ alkoxy, C₁₋₄ haloalkoxy, C₁₋₄ hydroxyalkyl, -O-(C₁₋₄ alkylene)-O-(C₁₋₄ alkyl), -C(=O)-NH-(C₁₋₄ alkylene)-N(C₁₋₄ alkyl)₂, or -C(=O)-O-(C₁₋₄ alkyl), alternatively, a plurality of R¹ and the atoms to which they are attached form a 5-7 membered ring;
R² is hydrogen, C₁₋₄ alkyl, or -(C₁₋₄ alkylene)-O-(C₁₋₄ alkyl);
X¹ is N or -CR⁶;
R⁶ is hydrogen, halogen, or C₁₋₄ alkyl;
R³ is hydrogen or C₁₋₄ alkyl, alternatively, R³ and R² together with the atoms to which they are attached form a 5-6 membered ring;
X² is N or -CR⁷;
R⁷ is hydrogen, halogen, C₁₋₄ alkyl, or C₁₋₄ haloalkyl;
X³ is N or -CR⁴;
R⁴ is hydrogen, halogen, or C₁₋₄ alkyl;
B is thienyl, thiazolyl, isothiazolyl, or thiadiazolyl;
each R⁵ is independently halogen, cyano, C₁₋₄ alkyl, C₁₋₄ haloalkyl, C₁₋₄ alkoxy, C₁₋₄ haloalkoxy, C₃₋₆ cycloalkyl, -C(=O)-O-(C₁₋₄ alkyl), phenyl, benzyl, pyridyl, -C(=O)-NH₂, or -NH-C(=O)-(C₁₋₄ alkyl), said phenyl, benzyl and pyridyl are optionally substituted with one or more substituents selected from halogen, cyano, amino, hydroxyl, C₁₋₄ alkyl and C₁₋₄ alkoxy;
m is 0, 1, 2, 3 or 4;
n is 0, 1, 2, or 3; and
said pharmaceutically acceptable form is selected from a pharmaceutically acceptable salt, a stereoisomer, a tautomer, a solvate, a N-oxide, and an isotopically labeled form.

2. The compound or the pharmaceutically acceptable form thereof according to claim 1, wherein
each R¹ is independently fluorine, chlorine, bromine, methyl, ethyl, isopropyl, trifluoromethyl, methoxy, ethoxy, isopropoxy, trifluoromethoxy, nitro, amino, -NH(CH₃), -NH(CH₂CH₃), -N(CH₃)₂, -N(CH₂CH₃)₂, -CH₂OH, -CH₂CH₂OH, -O-(CH₂)₂-OCH₃, -C(=O)-NH-(CH₂)₂-N(CH₃)₂, -C(=O)-NH-(CH₂)₃-N(CH₃)₂, -C(=O)-OCH₃, or -C(=O)-OCH₂CH₃.

3. The compound or the pharmaceutically acceptable form thereof according to claim 1 or 2, wherein
R² is hydrogen, methyl, ethyl, isopropyl, -(CH₂)₂-OCH₃, or -CH₂OCH₃, alternatively, R² and R³ together with the atoms to which they are attached form a 5-6 membered ring.

4. The compound or the pharmaceutically acceptable form thereof according to any one of claims 1-3, wherein
R⁶ is hydrogen, fluorine, chlorine, bromine, methyl, ethyl, or isopropyl.

5. The compound or the pharmaceutically acceptable form thereof according to any one of claims 1-4, wherein
R³ is hydrogen, methyl, ethyl, or isopropyl, alternatively, R³ and R² together with the atoms to which they are attached form a 5-6 membered ring.

6. The compound or the pharmaceutically acceptable form thereof according to any one of claims 1-5, wherein
R⁷ is hydrogen, fluorine, chlorine, bromine, methyl, ethyl, isopropyl, or trifluoromethyl.

7. The compound or the pharmaceutically acceptable form thereof according to any one of claims 1-6, wherein
R⁴ is hydrogen, fluorine, chlorine, bromine, methyl, ethyl, or isopropyl.

8. The compound or the pharmaceutically acceptable form thereof according to any one of claims 1-7, wherein
B is 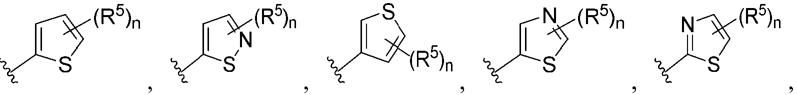 or

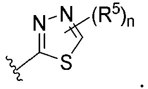

9. The compound or the pharmaceutically acceptable form thereof according to any one of claims 1-8, wherein
each R⁵ is independently fluorine, chlorine, bromine, cyano, methyl, ethyl, isopropyl, tert-butyl, cyclopropyl, cyclopentyl, cyclohexyl, trifluoromethyl, methoxy, ethoxy, isopropoxy, trifluoromethoxy, -C(=O)-OCH₃, -C(=O)-OCH₂CH₃, phenyl, benzyl, pyridyl, -C(=O)-NH₂, -NH-C(=O)-CH₃, or -NH-C(=O)-CH₂CH₃.

10. The compound or the pharmaceutically acceptable form thereof according to any one of claims 1-8 , being a compound of any one of formula IIa-1 to formula IIa-5 or a pharmaceutically acceptable form thereof: 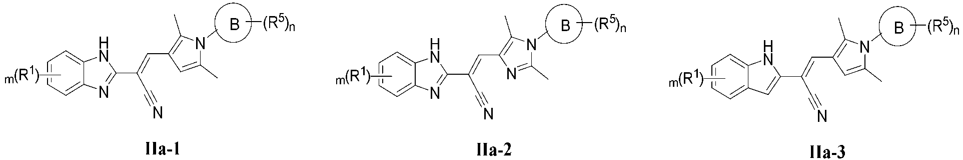 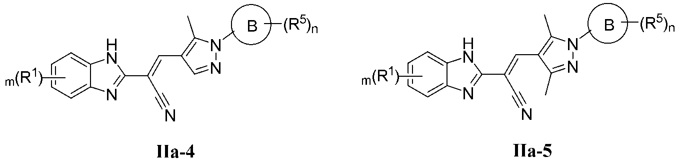 wherein B, R¹, R⁵, m and n are as defined in claim 1 or 2.

11. A compound or pharmaceutically acceptable form thereof selected from the group consisting of: 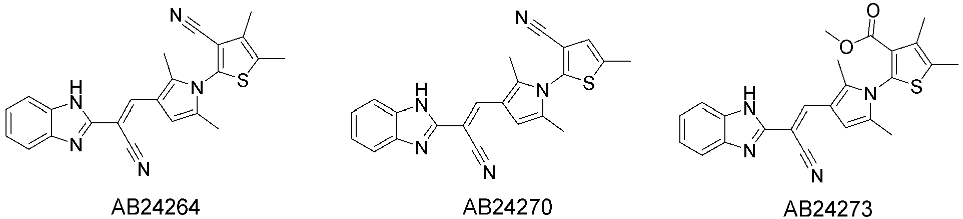 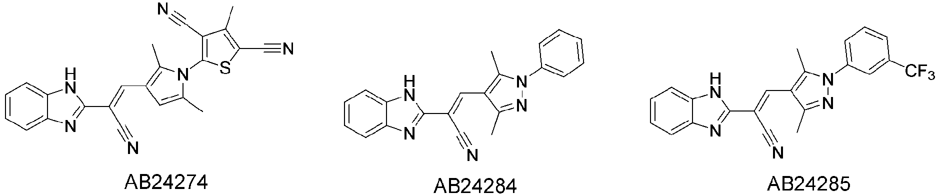 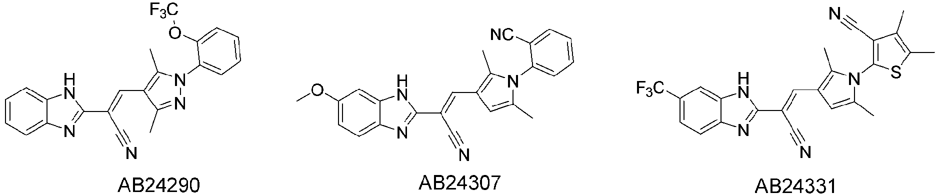 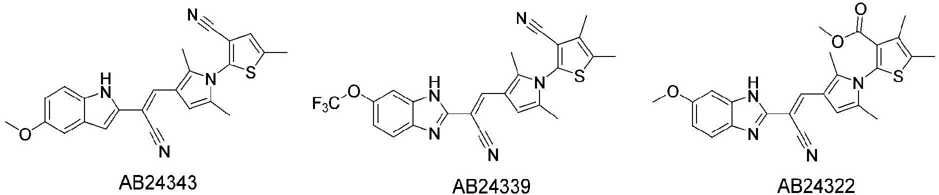 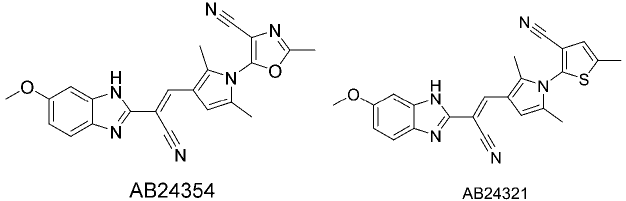 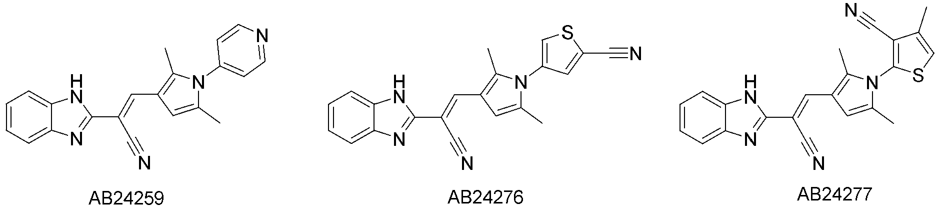 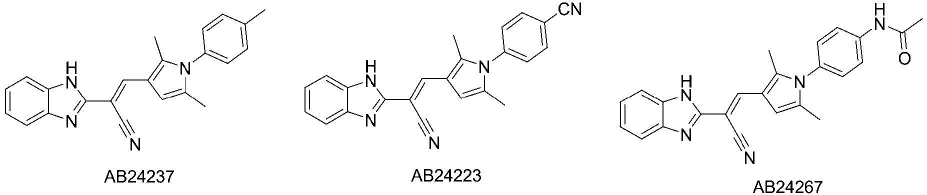 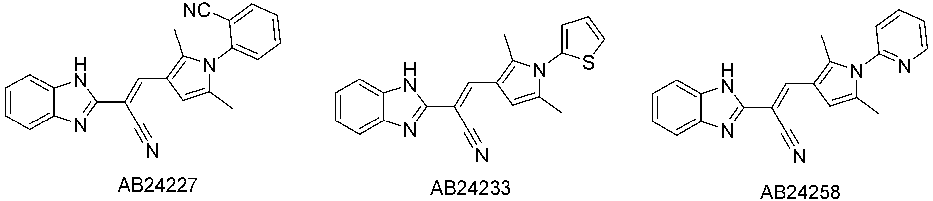 said pharmaceutically acceptable form is selected from a pharmaceutically acceptable salt, a stereoisomer, a tautomer, a solvate, a N-oxide, and an isotopically labeled form.

12. A pharmaceutical composition, comprising the compound or the pharmaceutically acceptable form thereof according to any one of claims 1 to 11 and one or more pharmaceutically acceptable carriers.

13. The compound or the pharmaceutically acceptable form thereof according to any one of claims 1 to 11 or the pharmaceutical composition of claim 12 for use in preventing and/or treating a disease or a disorder at least partially mediated by DHX33, the disease being selected from cancer, viral infection and inflammation that are mediated by DHX33.
